(19) Europäisches Patentamt
European Patent Office
Office européen des brevets

(11) Veröffentlichungsnummer : 0 232 507 B1

# (12) EUROPÄISCHE PATENTSCHRIFT

(45) Veröffentlichungstag der Patentschrift : **05.06.91 Patentblatt 91/23**

(51) Int. Cl.$^5$ : **A61B 5/11**

(21) Anmeldenummer : **86117130.4**

(22) Anmeldetag : **10.12.86**

## (54) Vorrichtung für die Untersuchung der Muskelkontraktion.

(30) Priorität : **11.12.85 DE 3543763**

(43) Veröffentlichungstag der Anmeldung : **19.08.87 Patentblatt 87/34**

(45) Bekanntmachung des Hinweises auf die Patenterteilung : **05.06.91 Patentblatt 91/23**

(84) Benannte Vertragsstaaten : **AT DE FR GB SE**

(56) Entgegenhaltungen :
**DE-A- 2 912 981**
**DE-A- 3 030 897**

(56) Entgegenhaltungen :
**MEDICAL AND BIOLOGICAL ENGINEERING AND COMPUTING, Band 19, Nr. 2, März 1981, Seiten 195-207, IFMBE, Stevenage, Herts, GB; S.W. JOHNSON et al.: "Identification of the stretch reflex using pseudorandom excitation: electromyographic response to displacement of the human forearm"**
**MEDICAL AND BIOLOGICAL ENGINEERING AND COMPUTING, Band 22, Nr. 5, September 1984, Seiten 458-460, IFMBE, Staines, Middlesex, GB; H. VERMARIEN et al.: "Mechanical impulse system for myotatic reflex investigation"**

(73) Patentinhaber : **Meyer, Niels**
**Adalbertstrasse 7a**
**W-6000 FRankfurt am Main (DE)**

(72) Erfinder : **Meyer, Niels**
**Adalbertstrasse 7a**
**W-6000 FRankfurt am Main (DE)**

(74) Vertreter : **Chambosse, Hans-Joachim**
**Dr.Jur.Rolf-S.Lehmpfuhl Rechtsanwalt und Notar Hans-Joachim Chambosse**
**Bockenheimer Landstrasse 63**
**W-6000 Frankfurt am Main (DE)**

EP 0 232 507 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Vorrichtung zur Bestimmung der Veränderung der mechanischen Größen bei der Muskel-kontraktion und zur Korrelation mit der Veränderung der elektrischen Größen von Nerv und Muskel bei der Muskelkontraktion, vorzugsweise am lebenden menschlichen Organismus. Auf verschiedenen wissenschaftlichen Gebieten, insbesondere in der Medizin, der Biologie und der Sportwissenschaft ist es erforderlich oder jedenfalls nützlich, bei der Untersuchung der Skelettmuskulatur die Veränderung der mechanischen Größen von Muskeln sowie der elektrischen Größen von Muskeln und den ihnen zugeordneten Nerven zu bestimmen und ihre Korrelation festzustellen. Dies ist zum Beispiel zur Beobachtung des Verlaufs von Rehabilitationsgrogrammen, Trainingsprogrammen im Sport der Entwicklung von Muskelerkrankungen (z.B. Muskeldystrophien) erforderlich. Ebenso sind diese Bestimmungen bei der Festlegung der Dosierung von muskelwirksamen Medikamenten oder auch in der Anästhesie angezeigt. Ferner gehört es zur medizinischen und biologischen Ausbildung, in den Bereichen Nervenkunde, Muskelmechanik, Reflexe und Elektromyographie, möglichst genaue Werte im vorgenannten Sinn bezüglich der Muskulatur bei der Muskelkontraktion zu erhalten.

Es sind zwar verschiedene Vorrichtungen und Verfahren für Muskeltests und zur wertmäßigen Ermittlung von Bewegungsvorgängen bzw. Muskelkräften bekanntgeworden. Diese Verfahren haben indessen durchweg erhebliche Nachteile. Insbesondere werden durchweg nur einzelne für die erforderlichen Bestimmungen maßgebliche Parameter bestimmt, obwohl erst durch die Feststellung und Korrelation sämtlicher Parameter (Nervensummenpotential, Nervenleitgeschwindigkeit, Muskelsummenpotential, Muskelvibrationen, Muskelkraft, Muskelweg und die verschiedenen Latenzzeiten) wissenschaftlich korrekte Ergebnisse zu erzielen sind.

Zudem haben die bekannten Vorrichtungen bzw. Verfahren schon die erheblichen Nachteile, daß eine ungenügende Fixierung der Bereiche des Körpers, insbesondere der Glieder, erfolgt, bezüglich deren die Muskelkontraktion untersucht werden soll. Der Proband kann desgleichen bei den bekannten Vorrichtungen und Verfahren auch nicht individuell in das Testgerät bzw. die Meßvorrichtung eingepaßt werden, obwohl dies angesichts der durchweg vorhandenen Unterschiede im Körperbau der einzelnen Menschen unabdingbar ist. Dadurch ist eine Verfälschung der anschließend bestimmten Werte verursacht, wie z.B. durch unterbleibende Erfassung eines Teilbereichs der Kontraktion und/oder Ausweichmöglichkeiten der untersuchten Glieder gegenüber der Meßvorrichtung.

Schließlich haben die bekannten Vorrichtungen bzw. Verfahren den Nachteil, daß die Ausgangswerte und Ausgangsbedingungen für die Unter-suchungen, insbesondere bezüglich Stromreizstärke, Stärke des Impulses bei Verwendung eines Reflexhammers, Größe der auf den jeweiligen Muskel ausgeübten Gegenkraft, nicht unveränderlich für den jeweiligen Versuch oder Versuchsreihen festgelegt werden können und eine weitere Fehlerguelle in Gestalt der Eigenreibung der Test- und Meßvorrichtung vorhanden sind.

Aus der Veröffentlichung "Identification of the stretch reflex using pseudorandom excitation" in Medical & Biological Engineering & Computing, Band 19, Nr. 2, 1981, Seiten 195-207, ist eine Vorrichtung bekannt, in der der Unterarm befestigt werden kann, um, von einem Zufallsgenerator ausgelöst, gestreckt zu werden. Mit Oberflächenelektroden werden die elektrischen Potentialschwankungen über der Beugeseite des Oberarms aufgenommen. Ferner ist aus der Veröffentlichung "Mechanical impulse system for myotatic reflex investigation" in Medical & Biological Engineering & Computing, Band 22, 1984, Seiten 458-460 eine Vorrichtung bekannt, bei der der sogenannte Patella-Sehnen-Reflex durch einen elektromagnetisch betriebenen und in Frequenz, Impulsdauer und Impulsstärke veränderlich auslösbaren Reflexhammer ausgelöst wird. Mit Oberflächenelektroden werden dabei die elektrischen Potentialschwankungen über dem musculus quadriceps femoris aufgenommen. Beide haben die Mehrzahl der vorgenannten Nachteile bekannter Verfahren und Vorrichtungen. Insbesondere sind die Verschienungseinheiten nicht genau und wiederholbar an die individuellen anatomischen Verhältnisse des Probanden anzupassen. Zudem kann nur jeweils eine Muskelgruppe des Unterarms untersucht werden. Bei dem in der Veröffentlichung in Medical & Biological Engineering & Computing Band 19 erwähnten Reflexhammer für die Auslösung der Muskelkontraktion können als weiterer Nachteil die Auftreffzeit und die Auftreffimpulsstärke des Reflexhammers nicht gemessen werden. Die beschriebene elektromagnetische Auslösung des Reflexhammers ist störanfällig und verteuert die Vorrichtung. Die in Medical & Biological Engineering & Computing Band 22 beschriebene Vorrichtung sieht eine Dehnungsvorrichtung für den Muskel vor, die jedoch keine unterschiedlichen Dehnungskräfte, Dehnungslängen und Dehnungsgeschwindigkeiten vorsieht.

Der Erfindung liegt die Aufgabe zugrunde, eine Vorrichtung der eingangs genannten Art zu schaffen, bei der unter Vermeidung der Nachteile der bekannten Verfahren und/ oder Vorrichtungen die Veränderung der mechanischen Größen unter Korrelation mit der Veränderung der elektrischen Größen von Nerv und Muskel bei der Muskelkontraktion zuverlässig und unter Ausschaltung von Fehlerquellen bestimmt werden können.

Diese Aufgabe wird erfindungsgemäß durch die im kennzeichnenden Teil des Patentanspruchs 1 aufgeführten Merkmale gelöst. Vorteilhafte Ausgestaltungen der Erfindung sind in den Unteransprüchen aufgeführt.

Es folgt die Beschreibung von Ausführungsbeispielen der Erfindung anhand von Zeichnungen. Dabei zeigen

Fig. 1 eine Isometrie-Darstellung der erfindungsgemäßen Vorrichtung für die Untersuchung der Muskelkontraktion mit fixiertem Unterschenkel und Fuß ;

Fig. 2 eine Aufsicht auf die Vorrichtung gemäß Fig. 1, jedoch vor der Fixierung des zu untersuchenden Glieds rechts des Muskelbereichs ;

Fig. 3 einen Längsschnitt durch die Vorrichtung gemäß Fig. 2 der Schnittstelle A-A ;

Fig. 4 als Detail eine alternative Ausgestaltung der Drehverstellung zwischen Basisplatte und Drehschemel in der Seitenansicht ;

Fig. 5 als Detail eine alternative Ausgestaltung der Drehverstellung wie in Fig. 4, jedoch in der Vorderansicht ;

Fig. 6 als Detail eine weitere Alternative der Drehverstellung zwischen der Basisplatte und dem Drehschemel der Vorrichtung ;

Fig. 7 als Detail den Drehschemel mit Basisplatte und an den Fuß zur Fixierung angesetzten Fühlern ;

Fig. 8 als Detail aus Fig. 3 den Reflexhammer der Vorrichtung ;

Fig. 9 als Detail aus Fig. 1 und 2 die Stütze für die Basisplatte nebst Führung in der Außenwand der Vorrichtung ;

Fig. 10 in Detaildarstellung die Ausgestaltung der erfindungsgemäßen Vorrichtung für die Untersuchung der Kontraktion der Armmuskulatur ;

Fig. 11 als Detail aus Fig. 1 den Stethoskopkopf mit Mikrophon zur Messung der Muskelvibration ;

Fig. 12 ein weiteres Ausführungsbeispiel der erfindungsgemäßen Vorrichtung mit Zugfeder als Gegenkraft und schneckenförmiger Walze in schematischer Darstellung ;

Fig. 13 eine weitere Ausführungsform der erfindungsgemäßen Vorrichtung mit druckluftgefülltem Behälter als Gegenkraft zur Muskelkontraktion ;

Fig. 14 als weitere Ausführungsform die angepaßte Vorrichtung zur Untersuchung der Muskelkontraktion des Daumenmuskels ;

Fig. 15 als Detail von Fig. 14 einen Schnitt an der Stelle C-C ;

Fig. 16 als weiteres Detail der Fig. 14 einen Schnitt an der Stelle D-D ;

Fig. 17 eine weitere Ausführungsform zur Untersuchung der Muskelkontraktion des Daumenmuskels in Vorderansicht ;

Fig. 18 als Detail von Fig. 17 die Handhalterung und Daumenschale mit eingelegter und fixierter Hand in Seitenansicht ;

Fig. 19 als weiteres Detail der Fig. 17 an der Schnittstelle E-E ;

Fig. 20 die in einem Bauteil zusammengefaßten Elektroden für das Elektromyogramm sowie Schalltrichter des Stethoskopkopfs mit nachgeschaltetem Mikrophon in der Ansicht von unten ;

Fig. 21 als Schnittzeichnung das Bauteil gemäß Fig. 20 in Seitenansicht an der Schnittstelle F-F.

Die erfindungsgemäße Vorrichtung für die Untersuchung der Muskelkontraktion besteht auf dem Gehäuse 1, das auf der Vorderseite, der Oberseite und der Unterseite offen ist, dem im Gehäuse eingesetzten Drehschemel 2 mit aufgesetzter Basisplatte 3 nebst oberer Gliedhalterung 4 und unterer Gliedhalterung 5 – im dargestellten Ausführungsbeispiel gemäß Fig. 1 und 2 zusätzlich der Fersenhalterung 6 – sowie den über die Achse 7 mit dem einen Schenkel des Drehschemels 2 verbundenen Elektromotor 9 mit Elektrokupplung 10 und dem über die Achse 8 mit dem anderen Schenkel des Drehschemels 2 verbundenen Potentiometer 11. In den Figuren 1 und 2 ist die erfindungsgemäße Vorrichtung in ihrer Anordnung zur Untersuchung der Kontraktion des Fußhebemuskels (musculus tibialis anterior) dargestellt, die jedoch auch zur Untersuchung der Wadenmuskulatur (Fußsenkung) dient. In dem Gehäuse 1 ist der Drehschemel 2 mittels der beiden Achsen 7 und 8, die jeweils im oberen Bereich der beiden vertikalen Schenkel des Drehschemels 2 angesetzt und jeweils durch die parallel zu ihnen verlaufende Außenwand 12 bzw. 13 des Gehäuses 1, vorzugsweise mit Kugellagern, geführt sind, in sagittaler Ebene drehbar eingesetzt. Auf dem Bodenstück des Drehschemels 2 ist die Basisplatte 3 zum Aufsetzen des Fußes mittels der Drehverstellung 14 höhenverstellbar und mittels eines Kugelkopfes in der Neigung veränderlich angebracht. Am hinteren linde der Basisplatte 3 ist vorteilhafterweise eine Fersenhalterung 6 vorgesehen, die über eine an sich bekannte lösbare Feststellvorrichtung in Längsrichtung der Basisplatte horizontal verschiebbar ist und die Ferse des jeweiligen Probanden fixiert.

Zur weiteren Fixierung und Vermeidung ungewollter Horizontal- und/oder Vertikalbewegungen des Fußes dient, wie in Fig. 1 dargestellt, die untere Gliedhalterung 5. Diese besteht in vorteilhafter Ausführungsform aus einer Schale aus thermoflexiblem Kunststoff, die der jeweiligen Form des Spanns des Fußes angepaßt und an der Basisplatte 3 befestigt wird.

Ebenso nicht erfindungsnotwendig, jedoch vorteilhaft ist unterhalb der Basisplatte 3 eine in einer kurvenförmig verlaufenden Ausnehmung 17 in der Außenwand 13 des Gehäuses 1 fest, jedoch lösbar und vertikal

verschiebbar angebrachte Stütze 16 vorgesehen, mit der die jeweils gewollte untere Endstellung der Basisplatte 3 und damit zugleich des Drehschemels 2 festgelegt wird.

An der Achse 7 ist an der Außenseite 12 des Gehäuses 1 die Elektrokupplung 10 und anschließend an diese der Elektromotor 9 angesetzt. Die Elektrokupplung besteht aus den beiden aneinandergrenzenden Scheiben 18 und 19, wobei zumindest die Kupplungsscheibe 19 einen Reib-/ Bremsbelag 20 hat. Der Kraftschluß der Kupplung sowie die Anpreßkraft der Scheiben 18 und 19 wird in an sich bekannter Weise durch einen Elektromagneten 21, 22 regelbar bewirkt, der von einer Stromquelle 23 gespeist wird. Die Kupplungsscheibe 19 ist über die Achse 7 mit dem Elektromotor 9 verbunden, der ebenfalls von der Stromquelle 23 gespeist wird und in seiner Drehzahl regelbar ist.

An der gegenüberliegenden Außenwand 13 des Gehäuses 1 ist an der Außenseite der Außenwand auf der Achse 8 der Potentiometer 11 angebracht.

Zur Fixierung des Unterschenkels des Probanden in der Vorrichtung ist, wie in Figur 1 bis 3 sichtbar, an der Rückwand 24 des Gehäuses 1 unter Zwischenschaltung einer Haltewand 25 die obere Gliedhalterung 4 vorgesehen die zur Anpassung an den Unterschenkel des des jeweiligen Probanden vertikal und/oder horizontal über bekannte Stellschrauben verstellbar ist und eine Bandage oder Halbschale zur Einspannung des Unterschenkels hat.

Der Elektromotor 9 ist weder an der Außenwand 12 des Gehäuses 1 noch an dem Gehäuse 26, das Elektrokupplung und Elektromotor umgibt, fest angeflanscht. Vielmehr ist die Achse 7 durchgeführt und in der Außenwand des Gehäuses 26 in einem Kugellager gelagert. Wie in Figur 2 dargestellt, sind an beiden Längsseiten des Elektromotors 9 horizontal verlaufende Biegebalken 27, 28 zum Gehäuse 26 mit Dehnungsstreifen 29, 30 befestigt. Statt dieser Biegebalken 27, 28 mit Dehnungsstreifen 29, 30 oder zusätzlich kann auf der Achse 8 zwischendem Vertikal- schenkel des Drehschemels 2 und dem Potentiometer 11 auch ein Torsionsmeßstreifen 31 aufgebracht sein.

Die Drehverstellung 14 hat, wie in Figur 3 dargestellt, eine Meßskala für die Festlegung bzw. die Feststellung des Abstands zwischen der Basisplatte und dem Bodenstück des Drehschemels 2. Eine andere Ausführungsform ist in Figuren 4 und 5 dargestellt. Dabei sind drei Paare korrespondierender und jeweils fest verbindbarer und wieder lösbarer Scheiben 32, 33, 34 vertikal übereinander angeordnet und miteinander über jeweils eine der Scheiben verbunden, wobei das obere Scheibenpaar 32 und das untere Scheibenpaar 34 in vertikaler Richtung, jedoch um 90° gegeneinander versetzt und das mittlere Scheibenpaar 33 horizontal angeordnet sind. An jedem der drei Scheibenpaar 32, 33, 34 sind Skalen 35, 36, 37 angebracht.

An den jeweils inneren, die vertikalen Schenkel des Dresehemels 2 durchdringenden Enden der Achsen 7 und 8 sind teleskopartig aus der Achse in horizontaler Richtung herausziehbare oder über eingesetzte Federn herausdrückbare Fühler 18 eingelassen. Diese werden bei der Vorbereitung der Untersuchungen mit der erfindungsgemäßen Vorrichtung an die höchsten Vorsprünge der Knöchel als Drehachse des oberen Sprunggelenks des Beins herangeführt. Danach wird über die Drehverstellung 14 sowie die Einstellung der Stütze 16 die Basisplatte 3 so Fixiert, daß die Drehachse dos Sprunggelenks in einer gedachten horizontalen Linie mit den Achsen 7 und 8 liegt.

Nach der Fixierung werden die Fühler 38 wieder in die Achsen 7 und 8 zurückgeführt.

In Figur 1 nur schematisch dargestellt, sind am Gehäuse 1 der erfindungsgemäßen Vorrichtung ferner ein über die Stromquelle 23 gespeister Reizstromstimulator 39, ein Verstärker 40 und ein Aufzeichnungsgerät 41 angebracht. An den Reizstromstimulator 39 sind wahlweise die Oberflächenelektroden 42 oder die Nadelelektroden 43 angeschlossen, über die der Muskel erregt wird.

Alternativ zu den Oberflächenelektroden 42 und den Nadelelektroden 43 kann, wie in Figur 2 und 8 dargestellt, zur Auslösung der Muskelkontraktion ein Reflexhammer 44 vorgesehen sein, der im dargestellten Ausführungsbeispiel gemäß Figur 2 und 3 an der Haltewand 25 im Bereich der Achillessehne angebracht ist. Er wird durch Spannung und Lösung einer Feder 45 mit einer Sperre 46 ausgelöst. Im Hammerkopf 47 befindet sich ein piezo-elektrisches Element 48, das je nach der Stärke des Anschlags des Reflexhammers 44 an Sehne bzw. Muskel einen unterschiedlich starken elektrischen Impuls gibt.

Zur Gewinnung der Meßwerte bei der Kontraktion des Muskels sind gemäß Figur 1 alternativ oder kumulativ die beiden Elektroden 49 und 50 zur Messung des Nervensummenpotentials und der Nervenleitgeschwindigkeit über dem Nerv des kontrahierten Muskels und die weiteren Elektroden 51 und 52 zur Messung der elektrischen Potentialschwankungen (EMG) über dem kontrahierten Muskel angebracht. Diese Elektroden sind mit dem Verstärker 40 verbunden. Daneben ist zur Messung der Muskelvibration über dem Muskel der Stethoskopkopf 53 mit nachgeschaltetem Mikrophon 54 angebracht, die ebenfalls mit dem Verstärker 40 verbunden sind.

Ebenso besteht eine Verbindung des Potentiometers 11 zum Verstärker 40 und eine Verbindung zwischen dem Reizstromstimulator 39 und dem Verstärker 40. Soweit der Reflexlammer 44 angebracht ist, wird dessen piezoelektrisches Element 48 gleichermaßen mit dem Verstärker verbunden. Schließlich besteht eine Verbindung zwischen dem Elektromotor 9 und dem Verstärker 40 zur Berücksichtigung von dessen jeweiligem Kraftpotential.

Die im Verstärker 40 verstärkten Impulse der vorgenannte Meßstellen bzw. Informationsgeber werden im angeschlossenen Aufzeichnungsgerät 41 während des Versuchsablaufs aufgezeichnet.

Mittels der beschriebenen Vorrichtung ergibt sich folgender Versuchsablauf : Nach Fixierung des Fußes und des Unterschenkels des Probanden in der erfindungsgemäßen Vorrichtung über die obere Gliedhalterung 4 und auf der Basisplatte 3 sowie Feststellung der Stütze 16 werden die Oberflächenelektroden 42 und/oder die Nadelelektroden 43 einerseits sowie die Elektroden 49, 50 und/oder 51/52 sowie der Stethoskopkopf 53 angelegt. Anschließend wird der Elektromotor 9 über die Stromquelle 23 mit einem vorbestimmten Strom auf eine konstante Drehzahl gebracht. Der Elektromotor 9 treibt die mit ihm festverbundene Scheibe 19 der Elektrokupplung 10 an. Durch Erregung des Elektromagneten 21, 22 mit einem vorbestimmten Strom über die Stromquelle 23 wird eine mehr oder weniger starke Verbindung der Kupplungsscheiben 18 und 19 durch Pressung, vorzugsweise mit Restschlupf hergestellt. Im dargestellten Ausführungsbeispiel wirkt die Rotationskraft im Uhrzeigersinn. Über die Achse 7 und den mit ihr verbunden vertikalen Schenkel des Drehschemels 2 wird dieser mit der Basisplatte und dem Fuß des Probanden im Zehenbereich nach vorne unten gedrückt, bis die Basisplatte 3 an der Stütze 16 anschlägt. Anschließend wird über die Stromquelle 23, den Reizstromstimulator 39 und die Elektroden 42 und/oder 43 oder über den Reflexhammer 44 der Muskel erregt und dadurch zur Kontraktion gebracht. Bei einer Muskelkontraktionskraft, die größer ist als die Reibungskraft zwischen den Kupplungsscheiben 18 und 19, bewegt sich der Fuß um die Drehachse des Sprunggelenks und die auf einer gedachten Linie mit ihr verlaufenden Achsen 7 und 8 mit dem Drehschemel 2 gegen den Uhrzeigersinn nach oben. Der Potentiometer 11 auf der Achse 8 mißt bei dieser Drehbewegung die Position der Achse im Verhältnis zum gesamten Bewegungsumfang und die Drehung der Achse bei der Kontraktion und gibt den gemessenen Wert über den Verstärker 40 an das Aufzeichnungsgerät 41. Gleichzeitig werden die von den Elektroden 49, 50 und/oder 51, 52 sowie vom Stethoskopkopf 53 gemessenen Werte über den Verstärker 40 zum Aufzeichnungsgerät 41 geleitet.

In Fig. 6 ist als Alternative zu der Drehverstellung 14 mit Meßskala eine Drehverstellung 14′ mit Kugelkopf vorgesehen. Zusätzlich sind an der Unterseite der Basisplatte 3 eine oder mehrere mit Skalen versehene, senkrecht zur Ebene der Basisplatte 3 verlaufende Meßstäbe 55 mit federnden Hülsen 56 angebracht, die bis zum Bodenstük des Drehschemels 2 reichen und nach der Fixierung des Unterschenkels und Fußes oder sonstigen Körperteils des Probanden mit der Drehverstellung 14′ die Abstände der Basisplatte 3 zum Bodenstück des Drehschemels 2 anzeigen.

In Fig. 10 ist die erfindungsgemäße Vorrichtung in ihrer Anordnung zur Untersuchung der Kontraktion des Oberarmmuskels dargestellt. Wie daraus ersichtlich ist, bedarf es dabei lediglich der Anpassung der oberen Gliedhalterung 4′ und der unteren Gliedhalterung 5′ an die unterschiedliche Anatomie des Arms bei im übrigen unveränderter Ausgestaltung der Vorrichtung.

Eine weitere Ausführungsform der erfindungsgemäßen Vorrichtung ist in Fig. 12 dargestellt. Anstelle des Motors 9 und der Kupplung 10 ist die Achse 7 an der Außenseite der Außenwand 12 mit einer schneckenförmigen Walze 57 und anschließender, senkrecht angeordneter Spiralzugfeder 58, die ihrerseits an einem horizontal verlaufenden, an der Außenwand 12 angebrachten Biegebalken 59 angebracht ist. Die schneckenförmige Walze hat vorzugsweise im Querschnitt die Form einer Kurve nach der Formel

$$r(\varphi) = r_0 \left(1 + 2 \frac{r_0}{x_0} \varphi\right)^{-\frac{1}{2}}$$

mit dem Spurfehlwindel $\psi'$

$$\cos\psi = \left(1 + 2 \frac{r_0 \cdot \varphi}{x_0}\right) \cdot \left[\left(1 + 2 \frac{r_0 \cdot \varphi}{x_0}\right)^2 + \left(\frac{r_0}{x_0}\right)^2\right]^{-\frac{1}{2}}$$

wobei $\varphi$ den Drehwinkel, $r_0$ den Ausgangsradius, $r(\varphi)$ den Radius nach Drehung der Walze um ein bestimmtes Winkelmaß über die Achse 7 und $x_0$ die Ausgangsvorspannung der Spiralzugfeder 58 bedeuten. Mittels der so ausgelegten schneckenförmigen Walze 57 wird die bei der Kontraktion des Muskels über die Drehung des Drehschemels 2 und der Achse 7 herbeigeführte Längung der Spiralzugfeder und die dabei steigende Zugkraft egalisiert und ein konstantes Drehmoment erreicht. Die Spiralzugfeder 58 mit einer nach Bedarf gewählten Zugkraft kann gegen andere gleicher Länge, jedoch mit größerer oder kleinerer Zugkraft ausgetauscht werden. Auch in diesem Fall bleibt durch die schneckenförmige Walze 57 das Drehmoment konstant. Die Spiralzugfeder 58 wirkt bei dieser Ausführungsform anstelle der Kombination von Elektromotor und Elektrokupplung als Gegenkraft gegen die Kraft des kontrahierten Muskels. Die bei der Muskelkontraktion über die Achse 7, die Walze 57 und die Spiralzugfeder 58 auf den Biegebalken 59 einwirkende Kraft wird wie bei dem zuvor beschriebenen Ausführungsbeispiel über den Verstärker 40 verstärkt und an das Aufzeichnungsgerät 41 weitergegeben. Die Verbindung zwischen dem unteren Ende der Spiralzugfeder 58 und der Walze 57 wird durch ein längenstabiles Material wie zum Beispiel Polyamiddraht hergestellt. In Weiterführung dieser Ausführungsform ist es auch möglich, mehrere Spiralzugfedern 58 mit einem Wechselgetriebe zu verbinden, das in einer Versuchsreihe in kurzen Abständen Kontraktionsversuche mit unterschiedlicher Gegenkraft der Spiralzugfedern durch geführt werden können.

In Fig. 13 ist als weitere Ausführungsform der erfindungsgemäßen Vorrichtung statt Elektromotor und Elektrokupplung 9 und 10 an der Achse 7 an der Außenseite der Außenwand 12 ein Zahnrad 60 angebracht, das in eine angepaßte Zahnschiene 61 eingreift, welche horizontal auf zwei oder mehreren Rollen 62 in beiden Richtungen verschiebbar ist. Diese Zahnschiene hat an ihrem einen Ende einen Kolben 63, der in einem angepaßten, zylinderförmigen Ausgang 64 eines Drucklufttanks 65 verschiebbar ist. Dieser Drucklufttank kann über die Zuführung 66 unter einen frei wählbaren Druck gesetzt werden, der über das Manometer 67 gemessen und über den Verstärker 40 zum Aufzeichnungsgerät 41 als Meßwert weitergegeben und aufgezeichnet wird. Wegen des relativ großen Volumens des Drucklufttanks 65 bleibt der in ihm herrschende, als Gegenkraft gegen den bei der Muskelkontraktion über die Achse 7, das Zahnrad 60, die Zahnschiene 61 und den Kolben 63 entstehenden Druck wirkende Innendruck praktisch konstant. Die übrige Ausgestaltung und Anordnung der Vorrichtung ist gegenüber der eingangs beschriebenen unverändert.

In den Figuren 14 bis 16 ist als weitere Ausführungsform die erfindungsgemäße Vorrichtung in ihrer Anpassung zur Untersuchung der Muskelkontraktion bei kleineren Gelenken und Muskeln am Beispiel des den Daumen in Richtung Finger ziehenden Muskels dargestellt. Die beiderseits des Daumen verlaufenden Achsen 68, die auf der gedachten Linie der Gelenkachse des Gelenks Articulatio carpometa-carpea pollicis liegen und durch das U-förmige Bauteil 72 verbunden sind, wird an den Endpunkten eines ebenfalls U-förmigen Bauteils 69 gelagert, das über die ausziehbare und dreidimensional drehbare Verstelleinrichtung 71 mit Meßskala an der Unterarmhalterung 70 befestigt ist. An dem. U-förmigen Bauteil 72 ist eine Leiste 73 mittels eines feststellbaren Gelenks 74 angebracht, die als Gliedhalterung wie die oben beschriebene Gliedhalterung 5 dient und bündig an die Oberfläche des Daumens in Ruhestellung angelegt wird. Der Daumen wird an der Leiste 73, zum Beispiel mittels schnellhärtender Starrbinden fixiert. Horizontal und im rechten Winkel zu der Achse 68 ist ein Hebel 75 angebracht, der in einer Führung 76 gelagert und darin in der Länge verschiebbar ist. Am entgegengesetzten Ende ist der Hebel 75 abgebogen ; an seinem äußersten Ende trägt er eine bewegliche Doppelrolle 77. Über diese Doppelrolle 77 drückt der Hebel 75 bei der Kontraktion des Daumenmuskels auf die Mittelplatte 78, die durch die Druckfeder 79 die Gegenkraft zur Muskelkraft erhält. Die Druckfeder 79 ist mit der Bodenplatte 80 als unteren Teil einer Rahmenkonstruktion fest verbunden, die aus der Bodenplatte 80, der Deckplatte 81 und den vier äußeren Streben 82 besteht. Durch die Bodenplatte 80 und die Deckplatte 81 verlaufen vier innere Streben 83, die jeweils zwischen in der Bodenplatte 80 und der Deckplatte 81 angebrachten Rollen geführt sind. Die Streben 83 sind mit der Mittel-platte 78 fest verbunden. Bewegt sich der Daumen durch Kontraktion des Muskels, so drehen sich die Achsen 68, wobei der Hebel 75 gegen die Federkraft der Druckfeder 79 nach unten bewegt wird. Zum Ausgleich der sich beim Zusammendrücken der Druckfeder 79 ändernden Federkraft bewegt sich die Doppelrolle 77 über ihre nach beiden Seiten verlängerte Drehachse in zwei Langlöcher 87. Diese sind in zwei gegenüberliegenden Seitenwänden 84 enthalten. In Fig. 15 ist eine dieser Seitenwände 84 mit Langloch 87 sichtbar. Die Druckfeder 79 ist durch Federn gleicher Länge, jedoch anderer Federkonstante auswechselbar, indem sie mit ihrem Tragezylinder 85 mit Bajonettverschluß gelöst wird. Bewegt sich der Hebel 75 nach unten oder oben, so wird die Doppelrolle 77 über ihre in die Langlöcher 87 eingreifenden Achsverlängerungen 86 in den Langlöchern 87 geführt. Dabei verschiebt sich der Hebel 75 in seiner Führung 76 und verändert damit die wirkende Hebellänge. Die Langlöcher 87 beschreiben eine mathematisch festgelegte Kurve, die vorteilhafterweise nach der Formel :

$$r(\varphi) = \frac{-y_0 + \sqrt{y_0^2 + 2\frac{M_0}{K}\varphi}}{\sin\varphi}$$

verläuft.

Dabei bedeuten r (φ) = wirkende Hebellänge als Verbindung des Achsdrehpunkts in der Führung 76 zum Ansatzpunkt der Doppelrolle 77 bei Drehung um den Winkel aus der Horizontalen ; r (φ = O) = r in horizontaler Lage ; $Y_o$ = Zusammendrückung der Feder bei r (φ = O) ; $M_o$ = über den gesamten Bereich konstantes Drehmoment ; k = Federkonstante.

In den Figuren 17 bis 19 ist in Fortentwicklung der Ausführungsform der erfindungsgemäßen Vorrichtung zur Untersuchung der Muskelkontraktion bei kleineren Gelenken und Muskeln, insbesondere des den Daumen in Richtung Finger ziehenden Muskels, der Daumen, wie in Fig. 18 sichtbar, in die Daumenschale 94 eingelegt und an dieser, zum Beispiel mittels schnellhärtender Starrbinden, fixiert. Die Daumenschale 94 ist über eine Halterung 95 mit einem an sich bekannten Feststellgelenk und den Bügel 93 mit der Achse 90 fest verbunden. Diese Achse 90 ist über zwei parallel innerhalb des Lagerhalterungsrohrs 92 angeordnete Kugellager 91 mit einem ebenfalls parallel zum Bügel 93 angeordneten Übersetzungsrad 113 verbunden und trägt an ihrem dem Bügel 93 entgegengesetzten Ende den Potentiometer 11. Zur angepaßten Lagerung und Fixierung der Hand des Probanden bei der Durchführung der Versuche ist die Handhalterung 97, 98, 99, 100 vorgesehen, die unterhalb der Daumenschale 94 und des Bügels 93 angeordnet ist. Die Handhalterung besteht aus dem Boden 97, auf dem die Handkante aufliegt, der am Boden 97 im rechten Winkel angebrachten und zur Anlegung der Handoberseite bestimmten Außenwand 98 sowie den beiden ebenfalls im rechten Winkel zum Boden 97 angeordneten, jedoch über verriegelbare Schienenführungen 101 und 102 in Richtung der Außenwand 98 verschiebbaren Außenwänden 99 und 100 zur Anlegung an die Handinnenseite. Die Handhalterung 97, 98, 99, 100 ist über eine am Boden 97 angebrachte Halterung mit skaliertem und feststellbarem Kugelgelenk 103, 103′ mit der skalierten Halterungsstange 105 verbunden, die wiederum mittels des skalierten feststellbaren Kugelgelenks 104, 104′ in der Bodenplatte 106 der Vorrichtung für die Untersuchung der Muskelkontraktion eingesetzt ist. Die Achse 90 mit dem Lagerhalterungsrohr 92 ist durch die im rechten Winkel auf der Bodenplatte 106 starr aufgesetzte Gehäusewand 108 geführt, wobei das Lagerhalterungsrohr 92 seinerseits starr mit der Gehäusewand 108 verbunden ist. Zur Vorbereitung der Untersuchung wird die Hand des Probanden in die Handhalterung 97, 98, 99, 100 eingelegt und über die Kugelgelenke 103, 104, Verschiebung des Halterungsstabs 105 über das Kugelgelenk 104 in vertikaler Richtung, Verschiebung der Außenwände 99 und 100 über die Schienenführungen 101 und 102 sowie die Peilstäbe 96 an der Achse 90 die Gelenkachse (Drehachse des Gelenks Articulatio carpometacarpea pollicis) mit der Achse 90 in einer (gedachten) Linie zur Deckung gebracht. Alsdann wird der Daumen in der Daumenschale 94 fixiert.

Das auf der Achse 90 an dem dem Bügel 93 entgegengesetzten Ausgang aus dem Lagerhalterungsrohr 92 angebrachte Übersetzungsrad 113 wirkt mit einem rechtwinklig zur Achse 90 angeordneten, jedoch nicht fest mit ihr verbundenen Stab 117 zusammen, der in Linearführungen 118 und 119, 120 in seiner Längsrichtung möglichst reibungslos beweglich ist. Dieser Stab 117 hat, wie in Fig. 19 dargestellt, in seinem dem Übersetzungsrad zugeordneten Bereich zwei Schnur- bzw. Drahthalterungen 115 und 116, mit einer Spannvorrichtung an mindestens einer der Halterungen, in denen eine im Gewinde des Übersetzungsrads 113 eingelegte Schnur (Draht) 114 mit ihren beiden Enden befestigt ist. Der Stab 117 ragt mit seinem einen Ende, wie in Fig. 17 erkennbar, in Längsrichtung beweglich in die Linearführung 118 hinein. An seinem anderen, der Linearführung 119, 120 nachgeordneten Ende ist eine elektrische Wicklung/Spule 123 mit einer Spulenhalterung 122 starr angebracht, die in einen Magnetkern 124 eingeführt ist bzw. eintaucht, jedoch darin ebenfalls in Längsrichtung des Stabs 117 mit diesem beweglich. Die elektrische Spule wird über die Stromguelle 23 und einen Stromregler 121 regelbar gespeist, so daß je nach der unterschiedlichen Stärke des aufgebauten Magnetfeldes der Stab 117 in seiner Längsrichtung in den Magnetkern 124 hineingezogen wird bzw. herausgedrückt wird.

Wird der untersuchte Muskel, ebenfalls über die Stromquelle 23 und den Reizstromstimulator 39 erregt und dadurch zur Kontraktion gebracht, so bewirkt er über die Daumenschale 94 und den Bügel 93 eine Drehbewegung der Achse 90 und damit des Übersetzungsrads 113. Diese Drehbewegung wird durch das Zusammenwirken des Übersetzungsrads 113, die Schnur/ Draht 114 und den Stab 117 in eine Längsbewegung des Stabs 117 umgeformt. Über die beschriebene Tauchmagnetspule 122, 123, 124 und den Stromregler 121 wird bei der Untersuchung eine der vom untersuchten Muskel auf den Stab 117 übertragenen Kraft entgegengerichtete Kraft ausgeübt, die in ihrer Charakteristik dabei jeweils bestimmbar und regelbar ist.

Die auf den Stab 117 wirkenden Kräfte werden über einen Zug/Druck-Aufnehmer 126, vorzugsweise mittels Piezokristalls oder Kondensators, registriert. Die Informationen über die vom kontrahierenden Muskel bewirkte Drehung der Achse 90 werden wie beim Ausführungsbeispiel gemäß Fig. 1 und 14 über den Potentiometer 11 registriert.

Die vom Zug-/Druck-Aufnehmer 126 und vom Potentiometer 11 registrierten Impulse werden wiederum wie bei den eingangs beschriebenen Ausführungsbeispielen im Verstärker 40 verstärkt und im angeschlossenen Aufzeichnungsgerät 41 zusammen mit den weiteren beschriebenen Parametern aufgezeichnet.

Für Versuche mit rein isometrischer Kontraktion ist in an sich bekannter Weise eine Fixierung/Verriegelung 125 des Stabs 117 für seine feste Einstellung in unterschiedlichen Ausgangspositionen vorgesehen ; vorzugsweise ist sie als Spulen-Magnetkernschalter ausgeführt und ebenfalls über die Stromquelle 23 und den Stromregler 121 einstellbar. Der Stab 117 mit den Linearführungen 118, 119, 120 und dem elektrischen Tauchmagneten 122, 123, 124 ist zweckmäßigerweise, wie in Fig. 17 bis 19 dargestellt, in der Versuchsapparatur derart angeordnet, daß der Magnetkern auf derselben Bodenplatte 106 wie die Handhalterung 97 bis 100 mit Haltestab 105 angebracht ist und die Linearführung 118 an einer in gleicher Ebene wie die Bodenplatte 106 führenden, rechtwinklig mit der Wand 108 fest verbundenen Deckplatte 112 fest verbunden ist. Nicht erfindungswesentlich, jedoch zweckmäßig können die verbleibenden offenen Seiten mit den Wänden 109, 110 und 111 versehen und damit ein geschlossenes Gehäuse gebildet werden.

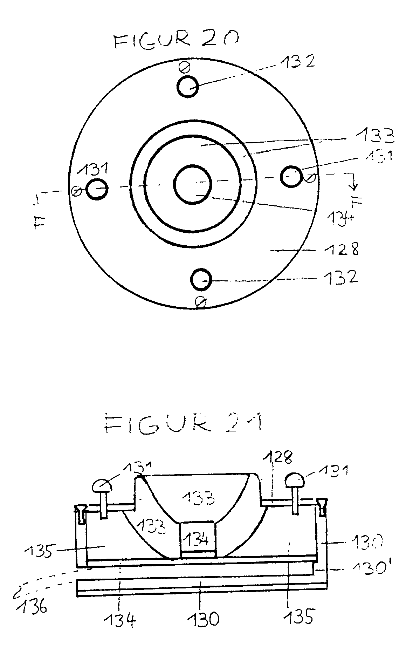

Schließlich ist in den Fig. 20 und 21 eine vorteilhafte Zusammenfassung der beschriebenen Elektroden 51 und 52 zur Messung der elektrischen Potentialschwankungen (EMG) und des Stethoskopkopfs 53 mit nachgeschaltetem Mikrophon 54 dargestellt. Der dem Stethoskopkopf 53 entsprechende Schalltrichter 133 ist in einem Gehäuse 130 mit einem offenen Deckel 128 eingesetzt. Dem Schalltrichter 133 nachgeschaltet ist das dem Mikrophon 54 entsprechende Mikrophon 134. Ebenfalls im Deckel 128, außerhalb des Durchtritts des Schalltrichters, sind zwei Differenzelektroden 131 und zwei Masseelektroden 132 des Elektromyographen befestigt, die den Elektroden 51 und 52 entsprechen. Die Elektroden 131, 132 sowie das Mikrophon 134 sind, wie beschrieben, mit dem Verstärker 40 und über diesen mit dem Aufzeichnungsgerät 41 verbunden. Es ist jedoch auch möglich, zur Verstärkung der von den Elektroden 131, 132 sowie der vom Mikrophon 134 ausgehenden Impulse unmittelbar im freien Innenraum 135 des Gehäuses 130 einen dem Verstärker 40 entsprechenden Verstärker anzuordnen, der mit dem Aufzeichnungsgerät 41 verbunden wird. Das vorstehend beschriebene Bauteil wird in entsprechender Weise wie bei der eingangs beschriebenen Anordnung über dem kontrahierenden Muskel angesetzt. Es kann darüber hinaus auch außerhalb der erfindungsgemäßen Vorrichtung für die Untersuchung der Muskelkontraktion zur gleichzeitigen Aufnahme von Elektrokardiogramm und Herzton über dem Herzmuskel eingesetzt werden.

Die erfindungsgemäße Vorrichtung bietet gegenüber den bekannten Vorrichtungen und Verfahren namentlich folgende Vorteile :

Sie ermöglicht erstmals die Erfassung sämtlicher maßgeblicher Parameter für die Bestimmung der Veränderung der mechanischen Größen bei der Muskelkontraktion und die Korrelation mit der Veränderung der elektrischen Größen von Nerv und Muskel bei der Muskelkontraktion.

Die Untersuchung der Muskulatur des Menschen und ebenso bei Tieren ist am lebenden Objekt und im intakten Funktionszusammenhang mit korrelativer Analyse des Zusammenhangs möglich. Insbesondere läßt sich damit vermeiden, wie bisher Aussagen über die menschliche Muskulatur nur durch Übertragung der Ergebnisse von Untersuchungen am Tier zu machen. In der Lehre kann auf die bisher allein verwendbaren Bioptate der Muskulatur getöteter Tiere verzichtet werden, und die Muskulatur kann bei der wissenschaftlichen Ausbildung in ihrem physiologischen Zusammenhang thematisiert und am eigenen Körper beobachtet werden.

Zudem ermöglicht die erfindungsgemäße Vorrichtung die Untersuchung der Muskulatur an nahezu allen Skelettmuskelfasern, Skelettmuskeln und Skelettmuskelgruppen ; die beschriebene Vorrichtung und das ihr zugrunde liegende Meßprinzip sind stets identisch. Es bedarf lediglich der Anpassung der Fixierungseinrichtungen für den jeweils untersuchten Körperbereich. Die mit der erfindungsgemäßen Vorrichtung für die Skelettmuskulatur erzielten Ergebnisse können wegen der erreichten wissenschaftlichen Zuverlässigkeit zum Beispiel auch auf Herzmuskulatur und Zwerchfellmuskulatur übertragen werden, was von besonderer Wichtigkeit für die pharmakologische Forschung und die Anästhesie ist.

Die Vorrichtung ermöglicht es, die Erregung des Muskels als Ausgangspunkt aller anschließenden Messungen auf eine definierte Größe zu bringen. Der Reiz für die Muskelerregung wird supramaximal ausgeführt, so daß äußere Einflüsse, die die Untersuchungsergebnisse verfälschen, entfallen. Es ist auch eine maximale Kontraktion des Muskels auslösbar und mit der Vorrichtung im eingangs genannten Sinn meß- und bestimmbar.

Die erfindungsgemäße Vorrichtung ermöglicht eine genaue, dem jeweiligen Versuchsobjekt angepaßte Fixierung und insbesondere, die Drehachsen des Drehschemels mit der Drehachse des untersuchten Körperteils (z.B. mit der Drehachse des oberen Sprunggelenks) als maßgebliche Voraussetzung genauer Untersuchungsergebnisse zur Deckung zu bringen. Durch die genaue Fixierung des untersuchten Körperteils werden die bei der Muskelkontraktion erzeugten Kräfte ohne Verzögerungsmoment oder sonstige Verlustquellen auf die Meßvorrichtungen übertragen. Stromreizstärke bzw. Stärke des Impulses des Reflexhammers und die Größe der auf den untersuchten Muskel über die Vorrichtung ausgeübten Gegenkraft sind genau und während der Untersuchung unveränderlich festlegbar. Die erzielten Meßergebnisse, insbesondere zu Nervensummenpotential, Nervenleitgeschwindigkeit, Muskelsummenpotential, Muskelkraft, Muskelweg, Muskelvibration und die verschiedenen Latenzzeiten, sind sämtlich quantifiziert, zusammen bestimmbar und somit korrelierbar. Die Vorrichtung ermöglicht es im Gegensatz zu den bekannten Vorrichtungen und Verfahren, die von ihr auf den Muskel ausgeübte Gegenkraft zu dosieren und über den Bewegungsbereich bei der Kontraktion des Muskels konstant zu halten. Dies ist von besonderer Bedeutung für die Aufnahme der den Muskel charakterisierenden

Unterstützungszuckungen und die Hill' sche Beziehung zwischen Kraft und Geschwindigkeit.

## Ansprüche

1. Vorrichtung zur Bestimmung der Veränderung der mechanischen Größen bei der Muskelkontraktion und zur Korrelation mit der Veränderung der elektrischen Größen von Nerv und Muskel bei der Muskelkontraktion, dadurch gekennzeichnet, daß der menschliche oder tierische Körperteil mit dem bei der Kontraktion zu untersuchenden Muskel direkt oder indirekt durch elektrische und/oder mechanische Impulse erregt wird und eine Mehrzahl von Meßstellen am untersuchten Körperteil angebracht und dieser in der Vorrichtung eingesetzt wird, wobei die Vorrichtung folgende Merkmale hat : eine in einem Gehäuse (1) der Vorrichtung mittels durch die Außenwände (12, 13) des Gehäuses führenden Achsen (7, 8) gelagerten Drehschemel (2), auf der der zu untersuchende Körperteil mit seinem durch die Kontraktion des Muskels bewegten Abschnitt, z.B. Fuß, angebracht wird, wobei die Achsen (7, 8) auf einer Linie mit der Drehachse des Gelenks des untersuchten Körperteils liegen ; Oberflächenelektroden (42), die über eine Stromquelle (23) und einen Reizstromstimulator (39) den Muskel an den relevanten Stellen des Körperteils erregen ; eine an der einen Achse (7) an der Außenseite (12) des Gehäuses (1) angesetzte Elektrokupplung (10) und anschließend an diese einen Elektromotor (9), wobei beide von der Stromquelle (23) regelbar gespeist werden und wobei die Elektrokupplung (10) aus aneinandergrenzenden Scheiben (18) und (19) besteht, zumindest die Kupplungsscheibe (19) einen Reib-/Bremsbelag (20) hat und die Kupplung durch einen Elektromagneten (21, 22) bedient wird ; ein auf der Achse (8) an der Außenseite der Außenwand (13) des Gehäuses (1) angebrachtes Potentiometer (11), sowie weiter Elektroden (49, 50) zur Messung des Nervensummenpotentials und der Nervenleitgeschwindigkeit über dem Nerv des kontrahierten Muskels und/oder Elektroden (51, 52) zur Messung der elektrischen Potentialschwankungen über dem kontrahierten Muskel und/oder einen Stethoskopkopf (53) mit nachgeschaltetem Mikrophon (54), die über dem Muskel angebracht sind, wobei die Elektroden (49, 50) bzw. (51, 52) sowie das Mikrophon (54) des Stethoskopkopfs (53), das Potentiometer (11) und der Elektromotor (9) mit einem Verstärker (40) und dieser mit einem Aufzeichnungsgerät für die Messwerte verbunden sind.

2. Vorrichtung gemäß Patentanspruch 1, dadurch gekennzeichnet, daß auf dem Bodenstück des Drehschemels (2) eine Basisplatte (3) für das durch den kontrahierenden Muskel zu bewegende Körperteil, zum Beispiel den Fuß, angebracht ist, das mittels einer Drehverstellung (14) höhenverstellbar und mittels eines Kugelkopfes auch in der Neigung veränderlich ist.

3. Vorrichtung gemäß Patentanspruch 1 und 2, dadurch gekennzeichnet, daß unterhalb der Basisplatte (3) eine in einer kurvenförmig verlaufenden Ausnehmung (17) in der Außenwand (13) des Gehäuses (1) fest, jedoch lösbar und vertikal verschiebbar angebrachte Stütze (16) als unterer Anschlag für die Basisplatte (3) vorgesehen ist.

4. Vorrichtung gemäß Patentanspruch 1 und 2, dadurch gekennzeichnet, daß auf der Basisplatte (3) eine Gliedhalterung (5) für das zu fixierende Körperteil angebracht ist, die vorteilhafterweise aus einer Schale aus thermoflexiblem Kunststoff zur Anpassung an die Form des jeweiligen Körperteils besteht.

5. Vorrichtung gemäß Patentanspruch 1, dadurch gekennzeichnet, daß anstelle der Oberflächenelektroden (42) zur Erregung des Muskels Nadelelektroden (43) vorgesehen sind.

6. Vorrichtung gemäß Patentanspruch 1, dadurch gekennzeichnet, daß statt der Oberflächenelektroden (42) zur Auslösung der Muskelkontraktion ein Reflexhammer (44) vorgesehen ist, der durch Spannung und Lösung einer Feder (45) mit einer Sperre (46) ausgelöst wird und in seinem Hammerkopf (47) ein piezo-elektrische Element (48) hat, das beim Anschlag des Hammers an Sehne bzw. Muskel einen elektrischen Impuls gibt.

7. Vorrichtung gemäß Patentanspruch 1 und 2, dadurch gekennzeichnet, daß die Drehverstellung (14) aus drei Paaren korrespondierender und jeweils fest verbindbarer und wieder lösbarer Scheiben (32), (33), (34) besteht, die vertikal übereinander angeordnet und miteinander über jeweils eine der Scheiben verbunden sind, wobei das Scheibenpaar (32) und das Scheibenpaar (34) in vertikaler Richtung, jedoch um 90° gegeneinander versetzt und das Scheibenpaar (33) horizontal angeordnet sind und daß an jedem der drei Scheibenpaare Skalen (35), (36), (37) angebracht sind.

8. Vorrichtung nach Patentanspruch 1 und 2, dadurch gekennzeichnet, daß am hinteren Ende der Basisplatte (3) eine Fersenhalterung (6) angebracht ist, die über eine lösbare Feststellvorrichtung in Längsrichtung der Basisplatte (3) zur Fixierung des aufliegenden Körperteils verschiebbar ist.

9. Vorrichtung gemäß Patentanspruch 1 und einem oder mehreren der Patentansprüche 2 bis 8, dadurch gekennzeichnet, daß an der Rückwand (24) des Gehäuses eine obere Gliedhalterung (4) angebracht ist, die über Stellschrauben vertikal und horizontal dem zu untersuchenden Körperteil angepaßt wird und diesen mittels Bandage, Halbschale usw. fixiert.

10. Vorrichtung gemäß Patentanspruch 1 und einem oder mehreren der Patentansprüche 2 bis 9, dadurch gekennzeichnet, daß an den jeweils inneren, die vertikalen Schenkel des Drehschemels (2) durchdringenden Enden der Achsen (7) und (8) teleskopartig aus der Achse herausziehbare oder über eingesetzte Federn herausdrückbaren Fühler (18) zur Einstellung der Basisplatte (3) über die Drehverstellung (14) auf die anatomischen Gegebenheiten des jeweiligen untersuchten Körperteils angebracht sind.

11. Vorrichtung gemäß Patentanspruch 1 und einem oder mehreren der Patentansprüche 2 bis 10, dadurch gekennzeichnet, daß anstelle des Elektromotors (9) und der Elektrokupplung (10) an der Außenseite der Außenwand (12) eine senkrecht angeordnete Spiralfeder (58) angebracht ist, die mit ihrem einen Ende über eine Verbindung aus längenstabilem Material mit einer auf der Achse (7) angebrachten schneckenförmigen Walze (57) zusammenwirkt und mit ihrem entgegengesetzten Ende an einem an der Außenwand (12) angebrachten Biegebalken (59) angebracht ist, wobei der Biege-balken mit dem Verstärker (40) und dem Aufzeichnungsgerät (41) verbunden ist.

12. Vorrichtung gemäß Patentanspruch 1 und einem oder mehreren der Patentansprüche 2 bis 10, dadurch gekennzeichnet, daß statt des Elektromotors (9) und der Elektrokupplung (10) an der Achse (7) an der Außenseite der Außenwand (12) des Gehäuses (1) über ein Zahnrad (60) und eine daran angepaßte Zahnschiene (61) einer Kolben (63) am einem Ende der Zahnschiene in einen Drucklufttank (65) und dessen dem Kolben angepaßten Ausgang (66) eingesetzt und darin verschiebbar ist.

13. Vorrichtung gemäß Patentanspruch 1 und einem oder mehreren der Ansprüche 2 bis 10, dadurch gekennzeichnet, daß der Elektromotor (9) keine starre Verbindung zum Gehäuse (1) und/oder dem Motorgehäuse (26) hat und die Achse (7) in der Außenwand des Motorgehäuses (26) in einem Kugellager gelagert ist und daß an beiden Längsseiten des Elektromotors (9) horizontal verlaufende Biegebalken (27), (28) zum Motorgehäuse (26) mit Dehnungsstreifen (29), (30) befestigt sind.

14. Vorrichtung gemäß Patentanspruch 1 und einem oder mehreren der Patentansprüche 2 bis 10, dadurch gekennzeichnet, daß auf der Achse (8) ein Torsionsmeßstreifen (31) aufgebracht. ist.

15. Vorrichtung gemäß Patentanspruch 1 und 2 sowie einem oder mehreren der Patentansprüche 3 bis 14, dadurch gekennzeichnet, daß die Basisplatte (3) eine Drehverstellung (14') mit Kugelkopf hat, wobei an der Unterseite der Basisplatte eine oder mehrere mit Skalen versehene, senkrecht zu ihr verlaufende Meßstäbe (55) mit federnden Hülsen (56) angebracht sind, die bis zum Bodenstück des Drehschemels (2) reichen.

16. Vorrichtung gemäß Patentanspruch 1, dadurch gekennzeichnet, daß an der Achse (7, 68) an der Außenseite (12) des Gehäuses (1) statt der Elektrokupplung (10) mit Elektromotor (9) eine Rahmenkonstruktion, bestehend aus der Bodenplatte (80), der Deckplatte (81) und den vier äußeren Streben (82), angebracht ist, in der parallel zu den äußeren Streben (82) durch Ausnehmungen in der Bodenplatte (80) und der Deckplatte (81) verlaufende Streben (83), die durch eine Mittelplatte (78) fest verbunden sind, verschiebbar sind, und daß die Mittelplatte (78) mit einem Hebel (75) über eine auf ihr aufliegende bewegliche Doppelrolle (77) zusammenwirkt, wobei der Hebel (75) mit der Achse (7, 68) über eine Führung (76) im rechten Winkel und in der Führung (76) verschiebbar verbunden ist und der Hebel (75) an seinem entgegengesetzten Ende im Bereich der Doppelrolle (77) über Achsverlängerungen (86) in zwei Langlöchern (87) der einander gegenüberliegenden Seitenwände (84) der Rahmenkonstruktion (80, 81,82) geführt wird, daß dabei die beiden Langlöcher (87) eine mathematisch festgelegte Kurve beschreiben und daß zwischen der Bodenplatte (80) und der Mittelplatte (78) eine zusammendrückbare Druckfeder (79) eingespannt ist.

17. Vorrichtung gemäß Patentanspruch 16, dadurch gekennzeichnet, daß die Druckfeder (79) mit ihrem unteren Ende im Bereich der Bodenplatte (80) in einem tassenförmigen Tragezylinder (85) eingesetzt ist, der mittels eines an sich bekannten Bajonettverschlusses an der Bodenplatte (80) fest, jedoch lösbar angesetzt ist, wobei die Druckfeder (79) durch eine entsprechende Öffnung der Bodenplatte (80) bis zur Mittelplatte (78) reicht und Druckfedern unterschiedlicher Federkonstante auswechselbar eingesetzt werden.

18. Vorrichtung gemäß Patentanspruch 16 und/oder 17, dadurch gekennzeichnet, daß auf dem Hebel (75) Dehnungsmeßstreifen (88) aufgebracht sind.

19. Vorrichtung gemäß Patentanspruch 1 oder 16, dadurch gekennzeichnet, daß statt des Gehäuses (1) mit dem drehbaren Drehschemel (2) und den durch die Außenwände (12, 13) des Gehäuses (1) der Vorrichtung führenden Achsen (7, 8) ein U-formiges Bauteil (72) mit den an seinen beiden entgegengesetzten Enden angebrachten Achsen (68) und eine an dem Bauteil (72) mittels eines Feststellgelenks (74) angebrachte Leiste (73) an dem zu untersuchenden Körperteil befestigt werden, wobei die Achsen (68) auf einer Linie mit der Drehachse des Gelenks des untersuchten Körperteils liegen und an den Endpunkten eines weiteren U-förmigen Bauteils (69) gelagert sind, das über eine ausziehbare und dreidimensional drehbare Verstelleinrichtung (71), an der eine Meßskala angebracht ist, an der in sich starren Halterung (70) für das zu untersuchende Körperteil befestigt ist.

20. Vorrichtung zur Bestimmung der Veränderung der mechanischen Größen bei der Muskelkontraktion und zur Korrelation mit der Veränderung der elektrischen Größen von Nerv und Muskel bei der Muskelkontraktion, dadurch gekennzeichnet, daß der menschliche oder tierische Körperteil mit dem bei der Kontraktion zu untersuchenden Muskel direkt oder indirekt durch elektrische und/oder mechanische Impulse erregt wird und eine Mehrzahl von Meßstellen am untersuchten Körperteil angebracht und dieser mit seinem durch die Kontraktion des Muskels bewegten Abschnitt, z.B. Daumen, in einer Schale (89) eingelegt und in dieser befestigt ist, während der nicht bewegte Abschnitt des Körperteils, z.B. die übrige Hand, in einer Halterung eingelegt und fixiert wird, wobei die Vorrichtung folgende Merkmale hat : die Halterung besteht aus einem Boden (97), einer daran im rechten Winkel angebrachten starren Außenwand (98) und Außenwänden (99, 100), die ebenfalls im rechten Winkel zum Boden (97) angeordnet und über verriegelbare Schienenführungen (101, 102) in in Richtung der Außenwand (98) verschiebbar sind ; die Halterung (97, 98, 99, 100) hat eine am Boden (97) angebrachte Halterung mit skaliertem und feststellbaren Kugelgelenk (103, 103') mit einer an der Kugel (103) fest angesetzten Halterungsstange (105), die ebenfalls skaliert und innerhalb eines weiteren Kugelgelenks (104, 104') längenverschiebbar ist, wobei das Kugelgelenk (104, 104') auf einer Bodenplatte (106) angebracht und auch seinerseits skaliert ist ; die Halterung (97, 98, 99, 100) wird mittels Verschiebung der Außenwände (99, 100) und mittels der Halterungsstange (105) sowie der Kugelgelenke (103, 103') und (104, 104') der Form des zu untersuchenden Körperteils angepaßt ; die Schale (94) ist über eine Halterung (95) mit einem bekannten Feststellgelenk und einen Bügel (93) mit einer Achse (90) fest verbunden, die innerhalb eines Lagerhalterungsrohrs (92) in zwei Kugellagern (91) gelagert ist, wobei das Lagerhalterungsrohr (92) starr mit der senkrecht zur Bodenplatte (106) verlaufenden Gehäusewand (108) verbunden ist, und wobei die Achse (90) und das Lagerhalterungsrohr (92) in das Gehäuse hineinreichen ; Peilstäbe (96) an beiden Ariden des Bügels (93) auf einer Linie mit der Drehachse des Gelenks des untersuchten Körperteils und der Achse (90) ; Oberflächenelektroden (42), die über eine Stromquelle (23) und einen Reizstromstimulator (39) den Muskel an den relevanten Stellen des Körperteils erregen ; auf der Achse (90) an der dem Bügel (93) entgegengesetzten Längsseite ein Übersetzungsrad (113) mit auf seinem Umfang angebrachtem Gewinde und anschließend ein Potentiometer (11) zur Messung der Drehbewegungskraft bei der Drehung der Achse (90), wobei das Übersetzungsrad (113) mit einem rechtwinklig zur Achse (90) angeordneten, jedoch nicht fest mit ihr und dem Übersetzungsrad (113) verbundenen Stab (117) zusammenwirkt, der in Linearführungen (118, 119, 120) in seiner Längsrichtung beweglich ist und in seinem dem Übersetzungsrad (113) zugeordneten Bereich zwei Schnur-/ Drahthalterungen (115, 116) mit einer Spannvorrichtung an mindestens einer der Halterungen hat, in denen im Gewinde des Übersetzungsgrads (113) eingelegte Schnur/ Draht (114) mit ihren beiden Enden befestigt ist ; der Stab (117) hat an seinem der Linearführung (119, 120) nachgeordneten Ende eine elektrische Wicklung (123) mit Spulenhalterung (122), die in einen getrennt an der Bodenplatte (106) angebrachten Magnetkern (124) eintaucht und über eine Stromquelle (23) und einen Stromregler (121) regelbar gespeist und je nach der Stärke des Magnetfeldes mit dem Stab (117) in den Magnetkern (124) hineingezogen oder herausgedrückt wird ; ein Zug-/Druck-Aufnehmer (126), der die auf den Stab (117) wirkende, vom kontrahierenden Muskel erzeugte und über den Bügel (93), die Achse (90) sowie das Übersetzungsrad (113) übertragene Kraft sowie die von der Tauchmagnetspule (122, 123, 124) ausgeübte bestimmte Gegenkraft registriert, wobei die registrierten Informationen wie die übrigen Informationen gemäß Patentansprusch 1 im Verstärker (40) verstärkt und im Aufzeichnungsgerät (41) aufgezeichnet werden ; eine Verriegelung (125) zur festen Einstellung des Stabs (117) an vorbestimmten Positionen seiner Längsrichtung, vorzugsweise als ebenfalls über den Stromregler (121) regelbarer Spulen-/Magnetkernschalter.

21. Vorrichtung gemäß Patentanspruch 1, 16 oder 20 und einem oder mehreren der Patentansprüche 2 bis 15, 17 bis 19, dadurch gekennzeichnet, daß die Elektroden zur Messung der elektrischen Potentialschwankungen (EMG) und der Stethoskopkopf mit nachgeschaltetem Mikrophon zur Messung der Muskelvibration derart in einem Gehäuse (130) zusammengefaßt sind, daß jeweils eine oder mehrere Differenzelektroden (131) und Masseelektroden (132) des Elektromyographen neben einem Schalltrichter (133) auf der als offener Deckel ausgestalteten Unterseite (128) des Gehäuses angebracht, ein Mikrophon (134) ebenfalls innerhalb des Gehäuses (130) dem Schalltrichter (133) nachgeschaltet ist und Verbindungen von den Elektroden (131, 132) sowie vom Mikrophon (134) zum Verstärker (40) und zum Aufzeichnungsgerät (41) vorgesehen sind.

## Claims

1. Device for the determination of the change in the mechanical magnitudes during muscular contraction and for the correlation with the change in the electrical magnitudes of nerve and muscle during muscular contraction, characterized in that the human or animal body part, with the muscle to be investigated during the contraction, is excited directly or indirectly by means of electrical and/ or mechanical impulses, and a plurality of measuring points are attached to the investigated body part, and the latter is inserted in the device, the device having following characteristic features : the body part to be investigated, with its portion, e.g. foot, which is

moved by means of the contraction of the muscle, is attached to a rotating bolster rotatable in the sagittal plane (2), which is supported in the housing (1) of the device by means of axles (7, 8) leading through the outer walls (12, 13) of the housing, wherein the axles (7, 8) lie on a line with the axis of rotation of the joint of the investigated body part ; surface electrodes (42), which excite the muscle by means of an electric current source (23) and a stimulus current stimulator (39), are attached to the places on the body relevant for muscular excitation ; an electric coupling (10) and an electric motor (9), which is connected with the latter, are connected to the one axle (7) on the outer side (12) of the housing (1), wherein both of these are fed in a controllable manner by the electric current source (23), and wherein the electric coupling (10) consists of disks (18) and (19), which adjoin one another, at least the coupling disk (19) has a friction/brake lining (20), and the coupling is operated by means of an electromagnet (21, 22) ; a potentiometer (11) is attached on the axle (8) at the outside of the outer wall (13) of the housing (1), and additionally electrodes (49, 50) for measuring the total nerve potential and the conductive speed of the nerve are attached above the nerve of the contracted muscle, and/or electrodes (51, 52) for measuring the electric potential fluctuations are attached above the contracted muscle, and/or a stethoscope head (53) with a microphone (54) connected downstream is attached above the muscle ; wherein the electrodes (49, 50) or (51, 52) and the microphone (54) of the stethoscope head (53), the potentiometer (11), and the electric motor (9) are connected with an amplifier (40), the amplifier (40) being connected with a recording device for the measured values.

2. Device according to patent claim 1, characterized in that a base plate (3) for the body part to be moved by means of the contracting muscle, for example the foot, is attached on the base piece of the rotating bolster (2), which body part is vertically adjustable by means of a rotating adjustment (14) and also changeable with respect to inclination by means of a spherical head.

3. Device according to patent claims 1 and 2, characterized in that a support (16) is provided below the base plate (3) as a lower stop for the base plate (3), which support (16) is attached in a recess (17) in the outer wall (13) of the housing (1) so as to be fixed, but detachable and vertically adjustable, the recess (17) extending in a curve-shaped manner.

4. Device according to patent claims 1 and 2, characterized in that a limb holder (5) for the body part to be fixed is attached on the base plate (3) and advantageously consists of a cup of thermoflexible plastic in order to adapt to the shape of the respective body part.

5. Device according to patent claim 1, characterized in that needle electrodes (43) are provided instead of the surface electrodes (42) for the excitation of the muscle.

6. Device according to patent claim 1, characterized in that a reflex hammer (44) is provided for triggering the muscular contraction, instead of the surface electrodes (42), the reflex hammer (44) being triggered by means of tensioning and relaxing a spring (45) with a catch (46) and having in its hammer head (47) a piezoelectric element (48) which transmits an electric impulse to tendon or muscle when the hammer strikes.

7. Device according to patent claims 1 and 2, characterized in that the rotational adjustment (14) consists of three pairs of corresponding disks (32), (33), (34), which can be securely connected and detached again and which are arranged vertically one above the other and are connected with one another by means of one of the disks in each instance, wherein the disk pair (32) and the disk pair (34) are arranged in the vertical direction, but so as to be offset by 90° relative to one another, and the disk pair (33) is arranged horizontally, and that scales (35), (36), (37) are attached at each of the three disk pairs.

8. Device according to patent claims 1 and 2, characterized in that a heel holder (6), which is displaceable in the longitudinal direction of the base plate (3) via a detachable fixing device in order to fix the body part placed on it, is attached at the rear end of the base plate (3).

9. Device according to patent claim 1 and one or more of patent claims 2 to 8, characterized in that an upper limb holder (4), which is adapted vertically and horizontally to the body part to be investigated by means of adjusting screws and fixes this body part by means of binding, half-shell, etc., is attached at the rear wall (24) of the housing.

10. Device according to patent claim 1 and one or more of patent claims 2 to 9, characterized in that feelers (18) for the adjustment of the base plate (3) to the given anatomical conditions of the respective body part to be investigated via the rotational adjustment (14) are attached at the respective inner ends of the axles (7) and (8), which ends penetrate the vertical legs of the rotating bolster (2), which feelers (18) can be pulled out of the axle in a telescopic manner or pressed out by means of inserted springs.

11. Device according to patent claim 1 and one or more of patent claims 2 to 10, characterized in that a vertically arranged spiral spring (58) is attached to the outside of the outer wall (12), instead of the electric motor (9) and the electric coupling (10), one end of the spiral spring (58) cooperates with a helical roller (57) attached on the axle (7) by means of a connection of material which is stable with respect to length, and its opposite end is attached to a flexure beam (59) attached at the outer wall (12), wherein the flexure beam is connected with the amplifier (40) and the recording device (41).

12. Device according to patent claim 1 and one or more of patent claims 2 to 10, characterized in that, instead of the electric motor (9) and the electric coupling (10) at the axle (7) at the outside of the outer wall (12) of the housing (1) via a toothed gear (60) and a toothed rail (61) adapted to the latter, a piston (63), at the one end of the toothed rail (61), is inserted in a compressed air tank (65) and its output (66), which is adapted to the piston, and is displaceable therein.

13. Device according to patent claim 1 and one or more of claims 2 to 10, characterized in that the electric motor (9) has no rigid connection with the housing (1) and/or the motor casing (26), and the axle (7) is supported in the outer wall of the motor casing (26) in a ball bearing, and that horizontally extending flexure beams (27), (28) are fastened to the motor casing (26) with stress measurement strips (29), (30) at both longitudinal sides of the electric motor (9).

14. Device according to patent claim 1 and one or more of patent claims 2 to 10, characterized in that a torque measuring strip (31) is attached to the axle (8).

15. Device according to patent claims 1 and 2 and one or more of patent claims 3 to 14, characterized in that the base plate (3) has a rotational adjustment (14') with spherical head, wherein one or more measuring rods (55), with resilient sleeves (56), provided with scales and extending perpendicularly relative to the base plate (3), are attached at the underside of the base plate (3) and extend until the base piece of the rotating bolster (2).

16. Device according to patent claim 1, characterized in that, instead of the electric coupling (10) with electric motor (9), a frame construction, consisting of the base plate (80), the cover plate (81) and the four outer struts (82), is attached to the axle (7, 68) at the outer side (12) of the housing (1), struts (83), extending parallel to the outer struts (82) through recesses in the base plate (80) and the cover plate (81) and securely connected by means of a centre plate (78), are displaceable in the frame construction (80, 81, 82) ; and that the centre plate (78) cooperates with a lever (75) by means of a movable double roller (77) lying on top of it, wherein the lever (75) is connected with the axle (7, 68) at a right angle via a guide (76) and so as to be displaceable in the guide (76), and the lever (75) is guided at its opposite end in two elongated holes (87) of the side walls (84) of the frame construction (80, 81, 82) in the area of the double roller (77) via axial elongations (86), the side walls (84) being located opposite one another, that the two elongated holes (87) thereby describe a mathematically determined curve, and that a compressible pressure spring (79) is tensioned between the base plate (80) and the centre plate (78).

17. Device according to patent claim 16, characterized in that the lower end of the pressure spring (79) is inserted in the area of the base plate (80) in a cup-shaped supporting cylinder (85), which is attached to the base plate (80) by means of a bayonet joint, known per se, so as to be fixed but detachable, wherein the pressure spring (79) extends through a corresponding opening of the base plate (80) until the centre plate (78), and pressure springs having different spring constants can be used interchangeably.

18. Device according to patent claim 16 and/or 17, characterized in that stress measurement strips (88) are attached to the lever (75).

19. Device according to patent claim 1 or 16, characterized in that, instead of the housing (1) with the rotatable rotating bolster (2) and the axles (7, 8) leading through the outer walls (12, 13) of the housing (1) of the device, a U-shaped structural component part (72), with the axles (68) attached to its two opposite ends, and a strip (73) attached to the structural component part (72) by means of a fixing joint (74), are fastened to the body part to be investigated, wherein the axles (68) lie on a line with the axis of rotation of the joint of the body part to be investigated and are supported at the end points of another U-shaped structural component part (69), which is fastened to the inherently rigid holder (70) for the body part to be investigated by means of an adjusting device (71), to which a measurement scale is attached and which can be pulled out and can be rotated in a three-dimensional manner.

20. Device for the determination of the change in the mechanical magnitudes and for the correlation with the change in the electrical magnitudes of nerve and muscle during muscular contraction, characterized in that the human or animal body part, with the muscle to be investigated during the contraction, is excited directly or indirectly by means of electrical and/or mechanical impulses, and a plurality of measuring points are attached to the investigated body part, and the latter with its portion which is moved by means of the contraction of the muscle, e.g. thumb, is placed in a cup (89) and fastened in the latter, while the portion of the body part which is not moved, e.g. the rest of the hand, is inserted and fixed in a holder, the device having following characteristic features : the holder consists of a base (97), a rigid outer wall (98), which is attached to the latter at a right angle, and outer walls (99, 100) which are likewise arranged at a right angle to the base (97) and are displaceable in the direction of the outer wall (98) by means of lockable rail guides (101, 102) ; the holder (97, 98, 99, 100) has a holder attached to the base (97) with graduated and fixable ball joint (103, 103') with a support bar (105), which is securely fixed on the ball (103) and is likewise graduated and longitudinally displaceable within a further ball joint (104, 104'), the ball joint (104, 104') being attached to a base plate (106) and also itself being

graduated ; the holder (97, 98, 99, 100) is adapted to the shape of the body part to be investigated by means of displacement of the outer walls (99, 100) and by means of the support bar (105) and the ball joints (103, 103') and (104, 104') ; the cup (94) is securely connected with a known fastening joint via a holder (95) and with an axle (90) via a stirrup (93), the axle (90) is supported within a bearing support pipe in two ball bearings (91), wherein the bearing support pipe (92) is rigidly connected with the housing wall (108) which extends vertically relative to the base plate (106), and wherein the axle (90) and the bearing support pipe (92) extend into the housing ; direction-finding rods (96) at the two ends of the stirrup (93) on a line with the centre of rotation of the joint of the investigated body part and the axle (90) ; surface electrodes (42), which excite the muscle by means of an electric current source (23) and a stimulus current stimulator (39) are attached to the places on the body part relevant for muscular excitation ; a translating gear wheel (113), which comprises a thread arranged on its circumference, and, following this, a potentiometer (11) for measuring the force of the rotational movement during the rotation of the axle (90) on the axle (90) at the longitudinal side opposite the stirrup (93), wherein the translating gear wheel (113) cooperates with a rod (117), which is arranged at a right angle to the axle (90), but is not connected with the latter or with the translating gear wheel (113) so as to be fixed, which rod (117) is movable in its longitudinal direction in linear guides (118, 119, 120) and has two cord/wire holders (115, 116) with a pretensioning device at at least one of the holders in the area assigned to the translating gear wheel (113), both ends of the cord/wire (114) inserted in the thread of the translating gear wheel (113) being fastened in the holders ; the rod (117) has an electric winding (123) with coil holder (122) at its end downstream of the linear guide (119, 120), the electric winding penetrating a magnet core (124), which is attached separately at the base plate (106), and being fed in a controllable manner by means of an electric current source (23) and a current regulator (121) and being pulled in or pressed out of the magnet core (124) with the rod (117) according to the intensity of the magnetic field ; the force which acts on the rod (117), which is generated by the contracting muscle and transmitted via the stirrup (93), the axle (90) and the translating gear wheel (113), as well as the determined counterforce exerted by the solenoid coil (122, 123, 124), are recorded by means of a tension/compression recorder (126), wherein the recorded data is amplified in the amplifier (40), like the other data according to patent claim 1, and is recorded in the recording device (41) ; a lock (125) is provided for the secure adjustment of the rod (117) in predetermined positions of its longitudinal direction, preferably as a coil/magnet switch, which is likewise controllable via the current regulator (121).

21. Device according to patent claims 1, 16 or 20 and one or more of patent claims 2 to 15, 17 to 19, characterized in that the electrodes for measuring the electric potential fluctuations (EMG) and the stethoscope head, with microphone connected downstream, for measuring the muscle vibration are combined in a housing (130) in such a way that one or more differential electrodes (131) and earth electrodes (132) of the electromyograph are attached, in each instance, next to a sound projector (133) on the underside (128) of the housing, which underside is constructed as an open cover, a microphone (134) is likewise connected downstream of the sound projector (133) within the housing (130), and connections are provided from the electrodes (131, 132) and from the microphone (134) to the amplifier (40) and to the recording device (41).

## Revendications

1. Appareil pour la détermination du changement des grandeurs mécaniques relatives à la contraction des muscles et pour la correlation avec le changement des grandeurs électriques du nerf et du muscle pendant la contraction du muscle. Ce dispositif est caractérisé comme suit : La partie du corps humain ou du corps de l'animal est directement ou indirectement excitée par des impulsions électriques et/ou mécaniques ; une majorité de points de mesure est installée sur la partie du corps examiné et cette dernière est insérée dans l'appareil qui a les caractéristiques suivantes ; une sellette pivotante (2) placée dans un boîtier (1) du dispositif à l'aide d'axes (7, 8) passant au travers des parois extérieures (12, 13) du boîtier ; sur cette sellette est placée la partie du corps à examiner ainsi que la section mise en mouvement par contraction du muscle, p. ex. le pied ; ici les axes (7, 8) se trouvent au même niveau que l'axe de rotation de l'articulation de la partie du corps examinée; des électrodes de surface (42) qui excitent le muscle aux sections significatives de la partie du corps à l'aide d'une source de courant (23) et d'un simulateur de courant excitateur (39) ; un dispositif d'embrayage électrique (10) placé à un axe (7) de la paroi extérieure (12) du boîtier (1), le tout lié à un moteur électrique (9) ; les deux sont chargés par la source de courant (23) et sont réglables. Le dispositif d'embrayage électrique (10) est constituai par deux disques (13) et (19) qui sont avoisinants ; au moins le disque du dispositif d'embrayage (19) possède une garniture de friction et de frein (20) et le dispositif d' embrayage est mis en marche par un électro-aimant (21, 22) ; un potentiomètre (11) placé sur l'axe du coté extérieur de la paroi extérieure (13) du boîtier (1) ainsi que d'autres électrodes (49, 50) utilisées pour mesurer le potentiel de l'ensemble des nerfs et la vitesse de conduction des nerfs, au-dessus du nerf du muscle contracté et/ou des électrodes (51, 52) pour mesurer

les fluctuations du potentiel électrique au-dessus du muscle contracté et/ou une tête de stéthoscope (53) avec microphone intercalé en arrière (54) qui sont placées au-dessus du muscle ; les électrodes (49, 50) ou (51, 52) ainsi que le microphone (54) de la tête du stéthoscope (53), le potentiomètre (11) et le moteur électrique (9) sont reliés à un amplificateur (40), lui-même relié à un appareil d'enregistrement des valeurs mesurées.

2. L'appareil suivant la revendication du brevet 1 est caractérisé comme suit : Une plaque de base (3) est placée sur le fond de la sellette pivotante pour la partie du corps, p.ex. le pied, à mettre en mouvement par la contraction du muscle, qui est déplaçable en hauteur à l'aide d'un mécanisme de réglage (14) et modifiable en inclinaison à l'aide d'une tête sphérique.

3. L'appareil suivant la revendication du brevet 1 et 2 est caractérisé comme suit : Au-dessus de la plaque de base (3), un support fixé (16) dans un creux de forme courbée (17) de la paroi extérieure (13) du boîtier (1), mais quand même détachable et verticalement déplaçable, est prévu comme arrêt inférieur pour la plaque de base (3).

4. L'appareil suivant la revendication du brevet 1 et 2 est caractérisé comme suit : Sur la plaque de base est placée une attache pour membres (5) destinée à la partie du corps à fixer, qui se constitue avantageusement d'une enveloppe/coque de matière plastique thermoflexible s'adaptant à la forme de la partie respective du corps.

5. L'appareil suivant la revendication du brevet 1 est caractérisé comme suit : Des électrodes d'aiguilles (43) sont prévues au lieu des électrodes de surface (42) pour l'excitation du muscle.

6. L'appareil suivant la revendication du brevet 1 est caractérisé comme suit : A la place des électrodes de surface (42) pour le déclenchement de la contraction musculaire un marteau à réflexes (44) est prévu. Ce marteau est actionné par la tension et le relâchement d'un ressort (45) par un arrêt (46) et possède à sa tête (47) un élément piézoélectrique (48) qui donne une impulsion électrique au moment du contact entre le marteau et le tendon ou le muscle.

7. L'appareil suivant la revendication du brevet 1 et 2 est caractérisé comme suit : Le mécanisme de réglage (14) est composé de trois paires de disques correspondants susceptible d'être liées de manière fixe et d'être détachées (32) (33) (34), et qui sont groupées les unes sur les autres verticalement et qui sont respectivement liées par l'un des disques ; ici, la paire de disques (32) et la paire de disques (34) sont groupées dans le sens vertical, mais placée de manière décalée à 90° l'une contre l'autre et la paire de disques (33) est positionnée de manière horizontale ; à chacune des trois paires de disques sont fixées des échelles (35) (36) (37).

8. L'appareil suivant la revendication du brevet 1 et 2 est caractérisé comme suit : Tout au bout de la plaque de base (3) une attache pour talon (6) est fixée qui est déplaçable en vue de la fixation de la partie du corps à déposer par un dispositif de blocage en direction longitudinale de la plaque de base (3).

9. L'appareil suivant la revendication du brevet 1 et une ou plusieurs des revendications du brevet 2 à 8 est caractérisé comme suit : Au dos (24) du boîtier, une attache pour membres supérieure (4) est fixée qui est adaptée verticalement et horizontalement par vis de fixation à la partie du corps à examiner et la fixe au moyen de bandages et de semi-enveloppe/coque etc.

10. L'appareil suivant la revendication du brevet 1 et une ou plusieurs des revendications 2 à 9 est caractérisé comme suit : Aux extrémités intérieures respectives des axes (7) et (8) qui transpercent les côtés verticaux de la sellette (2) sont placés des capteurs (18) qui sont retirables de l'axe de façon télescopique ou repoussables au moyen des ressorts installés, dans le but de l'ajustement de la plaque de base (3) aux données anatomiques de la partie du corps respective à l'aide du mécanisme de réglage (14).

11. L'appareil suivant la revendication du brevet 1 et une ou plusieurs des revendications 2 à 10 est caractérisé comme suit : Au lieu du moteur électrique (9) et de l'embrayage électrique (10) un ressort spiral (58) est installé verticalement sur la partie extérieure de la paroi extérieure (12) qui agit par l'une de ses extrémités, à l'aide d'une connexion de matériau de longeur constante, avec un cylindre de forme hélicoïde (57) placé sur l'axe (7). L'extrémité opposée de ce ressort est placée sur une poutre de flexion (59) installée à la paroi extérieure (12) ; la poutre de flexion est liée à l'amplificateur (40) et à l'appareil d'enregistrement (41).

12. L'appareil suivant la revendication du brevet 1 et une une ou plusieurs des revendications 2 à 10 est caractérisé comme suit : Au lieu du moteur électrique (9) et de l'embrayage électrique au niveau de l'axe (7) à la partie extérieure de la paroi extérieure (12) du boîtier (1), à l'aide d'une roue dentée (60) et une crémaillère adaptée (61), un piston (63) est placé à une extrémité de la crémaillière (61) dans un réservoir pneumatique (65) et sa sortie (66) est adaptée au piston, et y est déplaçable.

13. L'appareil suivant la revendication du brevet 1 et une ou plusieurs des revendications 2 à 10 est caractérisé comme suit : Le moteur électrique (9) n'a pas de liaison rigide avec le boîtier (1) et/ou avec le boîtier du moteur (26) ; l'axe (7) est situé dans un coussinet à billes de la paroi extérieure du boîtier du moteur (26) au niveau des deux côtés longitudinaux du moteur électrique (9) des poutres de flexion (27) (28) positionnées horizontalement sont attachées au boîtier du moteur (26) par des bandes de dilatation (29) (30).

14. L'appareil suivant la revendication du brevet 1 et une ou plusieurs des revendications du brevet 2 à

10 est caractérisé comme suit : Une bande de mesure de torsion (31) est placée sur l'axe (8).

15. L'appareil suivant la revendication du brevet 1 et 2 ainsi que d'une ou plusieurs des revendications du brevet 3 à 14 est caractérisé comme suit : La plaque de base (3) possède un mécanisme de réglage (14) avec tête sphérique ; au-dessous de la plaque de base une ou plusieurs règles graduées (55), ayant des échelles, avec douilles élastiques (56) sont positionnées verticalement par rapport à elle, et elles atteignent le fond de la sellette (2).

16. L'appareil suivant la revendication du brevet 1 est caractérisé comme suit : Au niveau de l'axe (7, 68), à l'extérieur (12) du boîtier (1), à la place de l'embrayage électrique (10) avec moteur électrique (9), une construction en cadre est installée, composée d'une plaque de fond (80), d'une plaque supérieure (81) et de quatre jambes de force extérieures (82) ; à l'intérieur de cette construction, les jambes de force positionnées parallèlement aux jambes de force extérieures (82), à l'aide de creux dans la plaque de fond (80) et la plaque supérieure (81), sont rigidement reliés par une plaque intermédiaire (78) et sont déplaçables. La plaque intermédiaire (78) agit avec un levier (75) par un rouleau double mobil (77) qui est placé sur elle ; le levier (75) est lié à l'axe (7, 68) par une glissière (76) dans l'angle droit et déplaçable dans la glissière ; le levier (75) est guidé à son extrémité opposée, dans le domaine du rouleau double (77), par allongements de l'axe (86), dans deux trous longitudinaux (87) des parois latérales opposées (84) de la construction en cadre (80, 81, 82) ; en même temps les deux trous longitudinaux (87) décrivent une courbe déterminée mathématiquement, et entre la plaque de fond (80) et la plaque intermédiaire (78), le ressort à pression compressible (79) est installé.

17. L'appareil suivant la revendication du brevet 16 est caractérisé comme suit : Le ressort à pression (79) est installé ; avec son extrémité inférieure, au niveau de la plaque de fond (80) dans un cylindre de suspension en forme de tasse (85), qui est lié à la plaque de fond (80) rigidement, mais détachable, au moyen d'une fermeture à baïonnette déjà connue connue en principe, le ressort à pression (79) atteint la plaque intermédiaire (78) par une ouverture correspondante de la plaque de fond (80) et des ressorts à pression de constante variable sont installés et ils sont échangeables.

18. L'appareil suivant la revendication du brevet 16 et/ou 17 est caractérisé comme suit : Des bandes de mesure de dilatation (88) sont installées sur le levier (75).

19. L'appareil suivant la revendication du brevet 1 ou 16 est caractérisé comme suit : Au lieu du boîtier (1) avec sellette pivotante (2) et les axes (7, 8) passant par les parois extérieures (12, 13) du boîtier (1) de l'appareil, il y a un élément en forme d'U (72) avec les axes (68) placés à ses deux extrémités opposées et un listeau (73) placé sur l'élément de construction (77) à l'aide d'une articulation de blocage (74) ; tous les deux sont fixés à la partie du corps à examiner. Ici les axes (68) se trouvent au même niveau que l'axe de rotation de l'articulation de la partie du corps à examiner et aux points extrêmes d'un autre élément de construction en forme d'U (69) qui est fixé à l'attache, rigide en elle-même, (70) pour la partie du corps à examiner au moyen d'un dispositif de réglage (71) qui est extensible et rotatif de manière tridimensionnelle et à laquelle est placée une échelle de mesure.

20. Appareil pour la détermination du changement des grandeurs mécaniques au moment de la contraction musculaire et pour la correlation avec la modification des grandeurs électriques du nerf et du muscle au moment de la contraction du muscle ; cet appareil est caractérisé comme suit : La partie du corps humain ou animal ainsi que le muscle à examiner au moment de la contraction est excité directement ou indirectement par des impulsions électriques et/ou mécaniques. Plusieurs points de mesure sont installés au niveau de la partie du corps examinée et cette dernière ainsi que la section mise en mouvement par la contraction musculaire, comme p.ex. le pouce, sont mises dans l'enveloppe/la coque (89) et sont fixées, alors que la section de la partie du corps non mise en mouvement, comme p.ex. le reste de la main, est placée, et fixée dans une attache ; ce dispositif a les caractéristiques suivantes : L'attache est composée d'un fond (97), d'une paroi extérieure rigide (98) placée au fond dans l'angle droit, et de parois extérieures (99, 100) qui sont également groupées dans l'angle droit sur le fond (97) et sont déplaçables vers la paroi extérieure (98) au moyen de glissières de guidage blocables (101, 102). L'attache (97, 98, 99, 100) possède une attache placé sur le fond (97) avec articulation sphérique graduée et blocable (103, 103') avec une barre d'attache (105) rigidement fixée à la bille (103) qui est également graduée et dont la longueur est modifiable dans une autre articulation sphérique (104, 104') ; cette dernière (104, 104') est montée sur une plaque de fond (106) et est elle-même graduée. L'attache (97, 98, 99, 100) est adaptée à la forme de la partie du corps à examiner par déplacement des parois extérieures (99, 100) et au moyen de la barre d'attache (105) ainsi que des articulations sphériques. L'enveloppe/la coque (94) est rigidement liée à un axe (90) par une attache (95) avec articulation de blocage connue et arceau (93); l'axe est situé dans deux roulements à billes (91) à l'intérieur d'un tuyau spécifique maintenant le roulement (92). Ce dernier est rigidement lié à la paroi du boîtier (108) placée verticalement par rapport à la plaque de fond (106). L'axe (90) et le tuyau spécifique maintenant le roulement (92) conduisent à l'intérieur du bottier ; des barres de relèvement (96) aux deux extrémités de l'arceau (93) au même niveau que l'axe de rotation de l'articulation de la partie du corps examinée et que l'axe (90) ; des électrodes de surface (42) qui excitent le

muscle aux points significatifs de la partie du corps grâce à une source de courant (23) et un simulateur de courant excitateur (39) ; au niveau de l'axe (90) sur le côté longitudinal opposé à l'arceau (93), il y a une roue de translation (113) ayant un filet sur la partie extérieure ; et ensuite il y a un potentiomètre (11) pour mesurer la force de rotation au moment de la rotation de l'axe (90) ; ici la roue de translation (113) agit avec la barre (117) qui est placée dans l'angle droit par rapport à l'axe (90), qui cependant n'est pas fixée rigidement à elle et à la roue de translation ; cette barre est mobile dans le sens de la longueur dans les glissières de guidage (118, 119, 120) linéaires, et elle possède dans son domaine lié à la roue de translation (113) deux attaches à ficelle/à fil (115, 116) avec un dispositif de tension au moins au niveau d'une des attaches dans lesquelles une ficelle/un fil (114) est fixé par ses deux bouts dans le filet de la roue de translation (113) ; la barre (117) possède un enroulement électrique (123) avec attache de bobines (122) au niveau de son extrémité placée agrès la glissière de guidage linéaire ; cet enroulement est plongé dans un noyau d'un aimant (124) placé séparément au niveau de la plaque de fond (106), et il est chargé de, façon réglable par une source de courant (23) et un régulateur de courant (121) et peut, grâce à la barre (117), être attiré dans le noyau d'un aimant (124) ou repoussé selon l'intensité du champs magnétique. Un capteur/récepteur de traction/pression (126) qui enregistre la force agissant sur la barre (117) et résultant de la contraction musculaire, transmis par l'arceau (93), l'axe (90) de même que la roue de translation (113) ainsi que la contre-force qui est déterminée par la bobine à aimant plongeur (122, 123, 124). Les informations enregistrées de même que les autres informations sont amplifiées selon la revendication du brevet 1 dans l'amplificateur (40) et sont enregistrées dans l'appareil d'enregistrement (41) ; un système de blocage (125) permettant le positionnement fixe de la barre (117) aux positions prédéterminées dans son sens longitudinal, de préférence sous forme d'un interrupteur à bobine/noyau d'un aimant également réglable par le régulateur d'intensité de courant (121).

21. L'appareil suivant la revendication du brevet 1, 16 ou 20 et une ou plusieurs des revendications 2 à 15, 17 à 19, est caractérisé comme suit : Les électrodes pour mesurer les fluctuations du potentiel électrique et la tête du stéthoscope avec microphone liée pour mesurer la vibration musculaire sont regroupés dans le boîtier (130) de sorte : qu'une ou plusieurs électrodes respectives de différence (131) et des électrodes de masse (132) du électro-myographe soient placées sur la partie inférieure du boîtier conçue comme un couvercle ouvert (128) ; qu'un microphone (134) soit subordonné au pavillon (133) également à l'intérieur du boîtier (130); et que des liaisons soient prévues, partant des électrodes (131, 132) de même que du microphone (134), en direction de l'amplificateur (40) et de l'appareil d'enregistrement.(41)

FIGUR 1

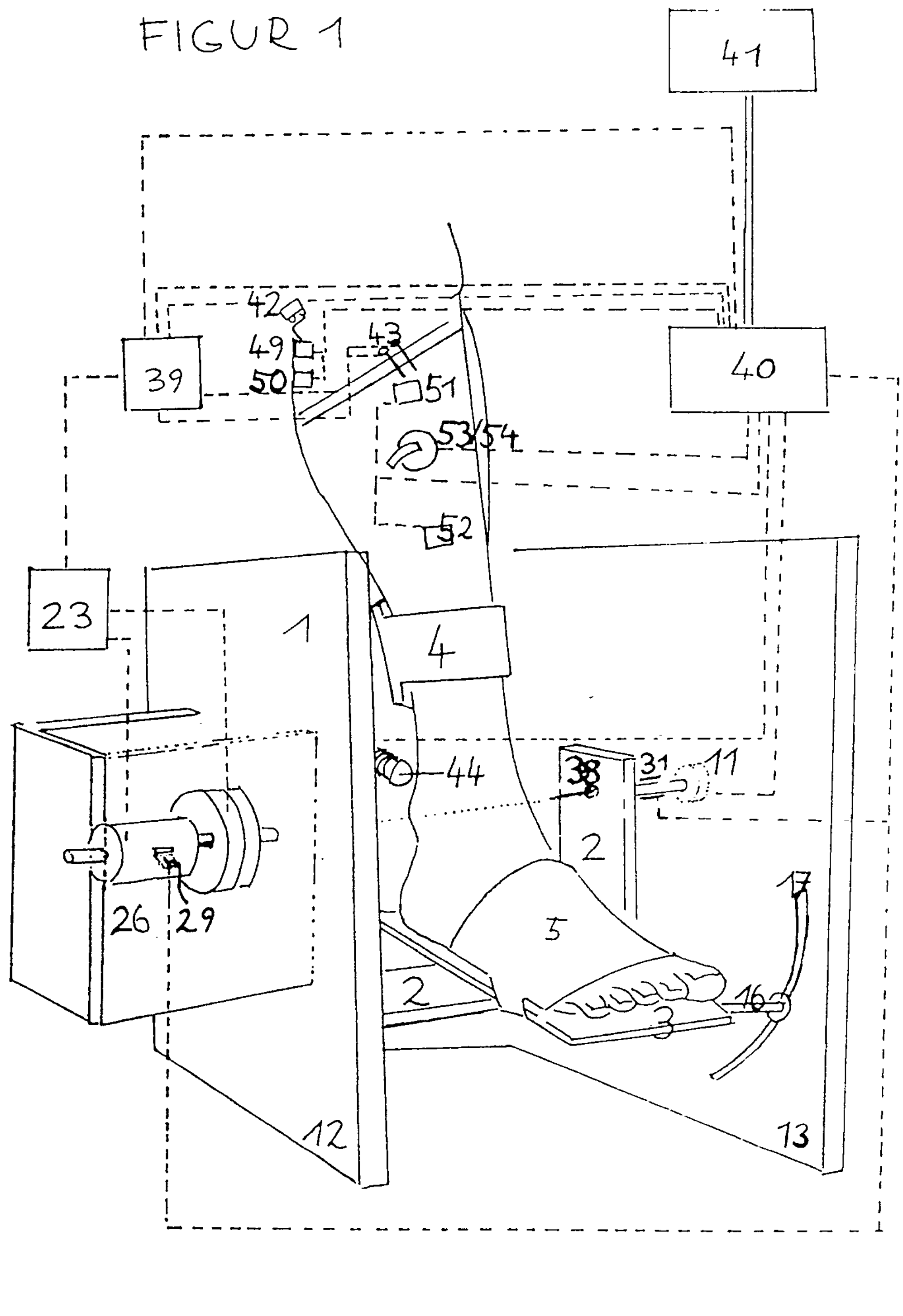
41
40
39
42
49
50
43
51
53/54
52
23
1
4
44
38
31
11
2
26
29
5
2
16
3
17
12
13

FIGUR 2

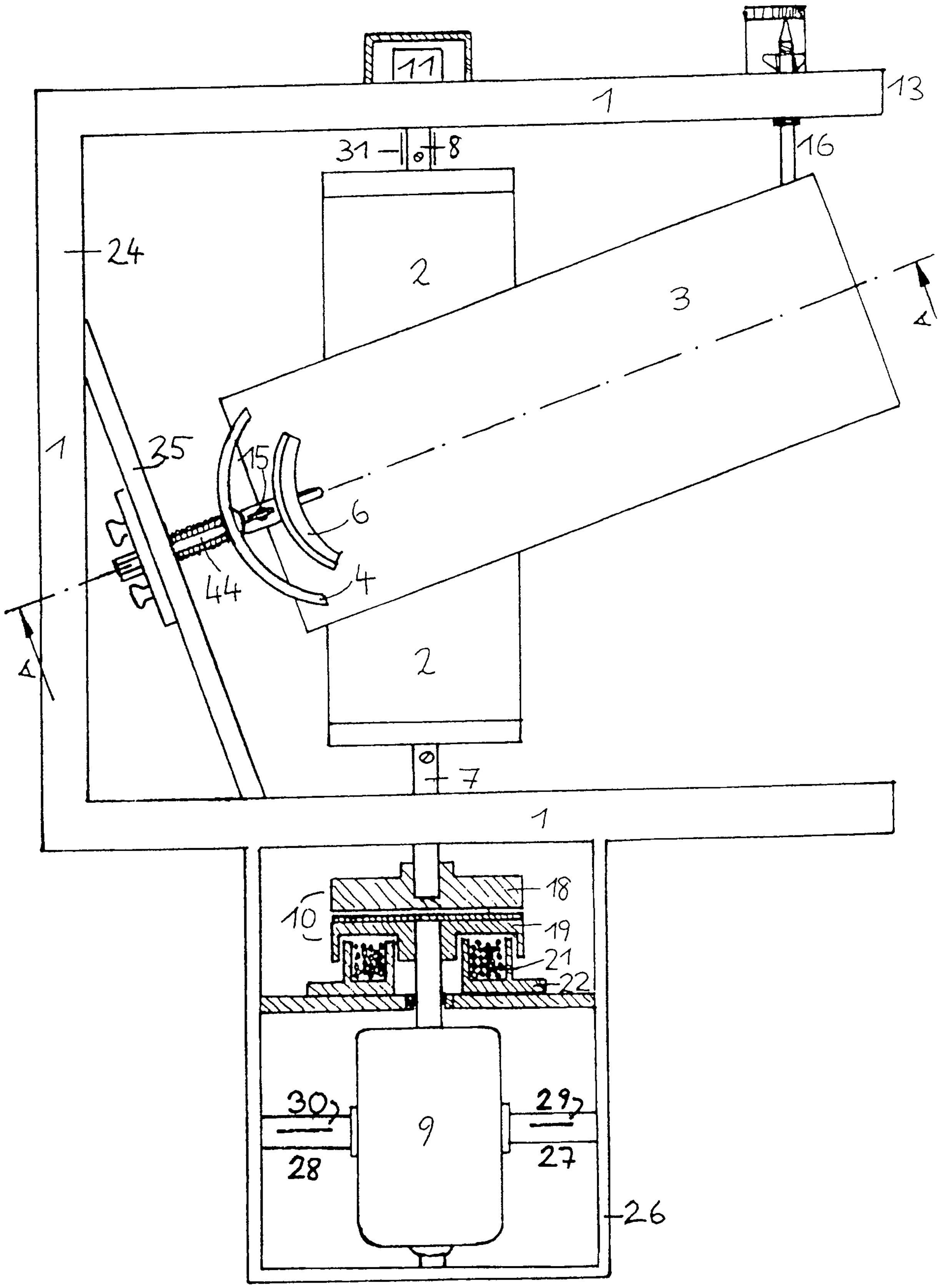

FIGUR 3

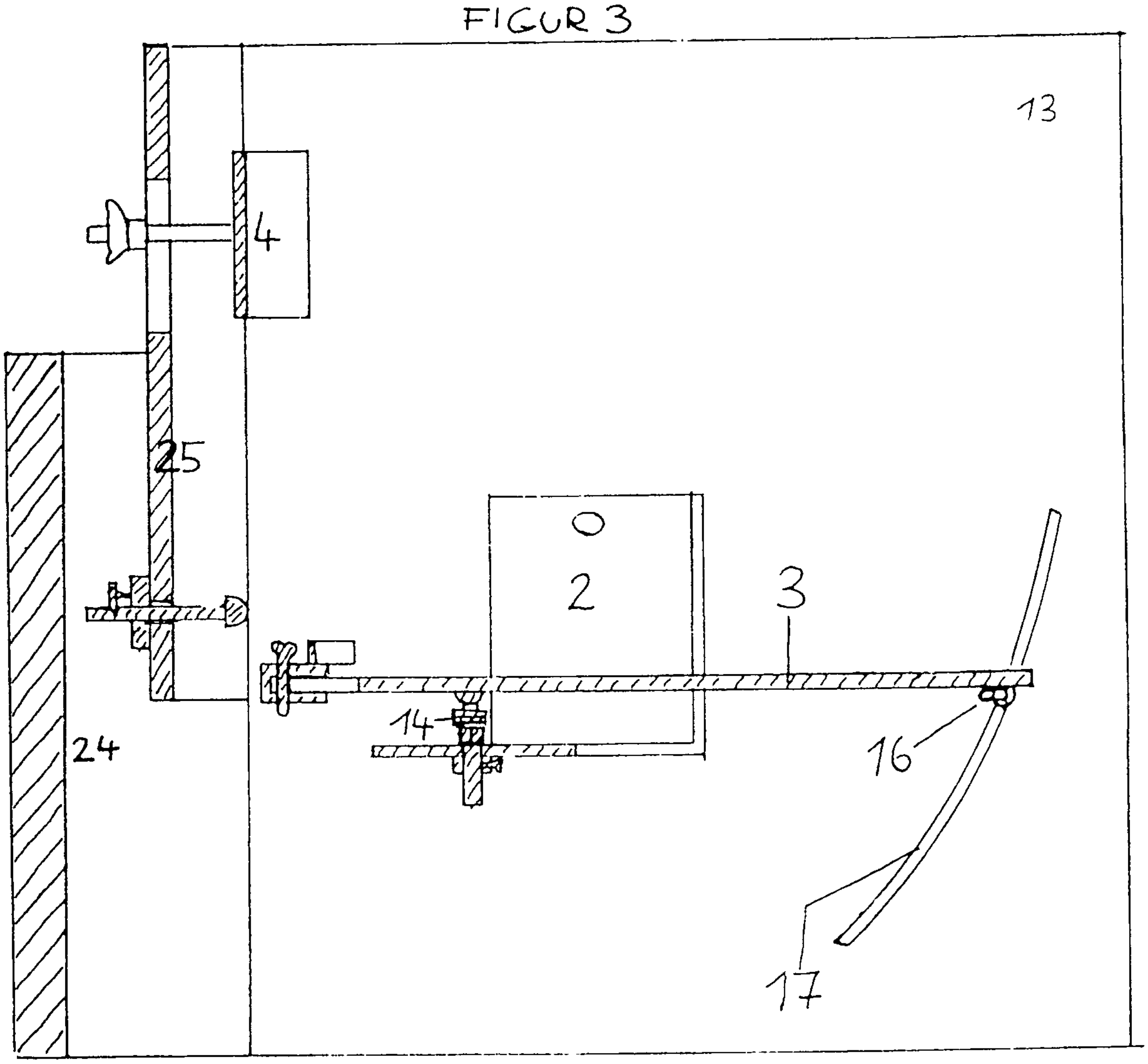

FIGUR 4

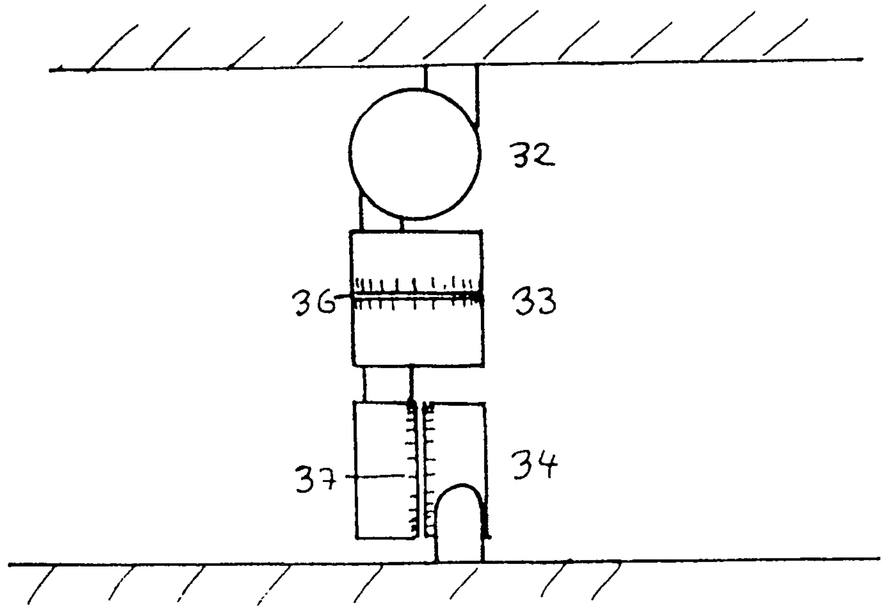


FIGUR 5

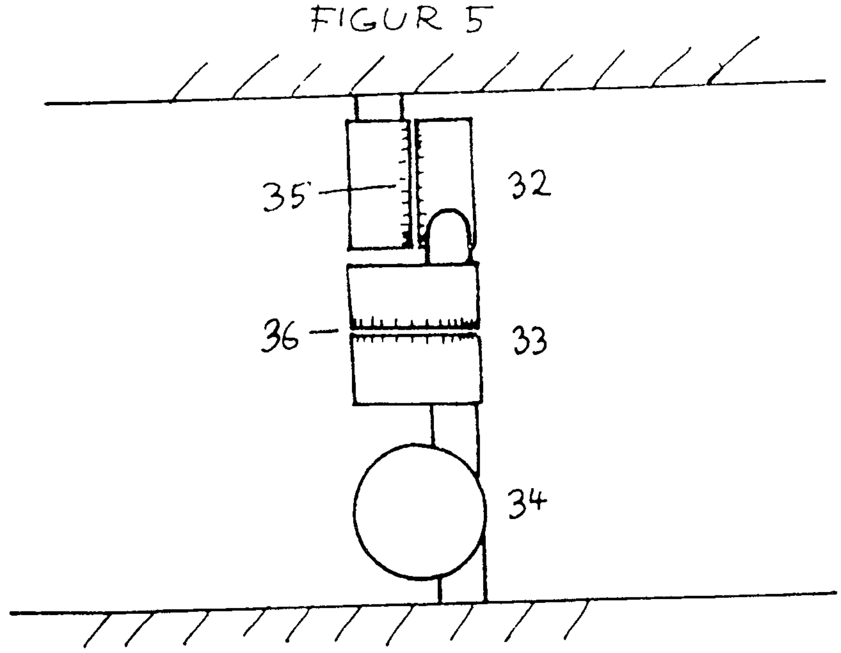

FIGUR 6

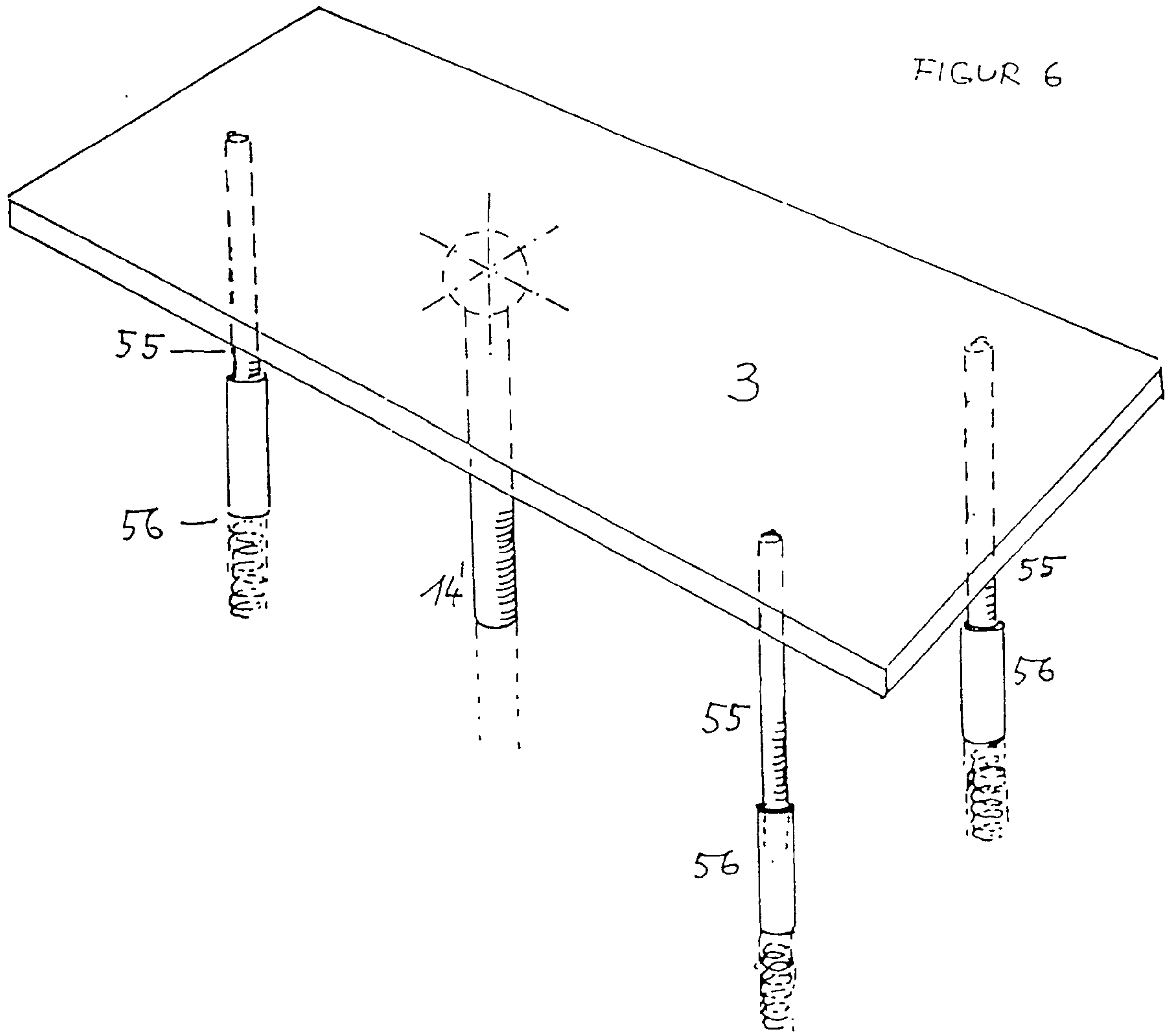

FIGUR 11

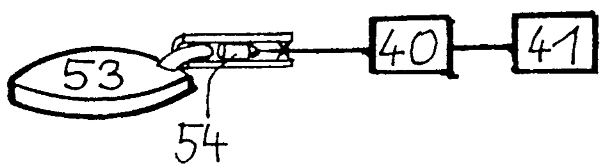


FIGUR9

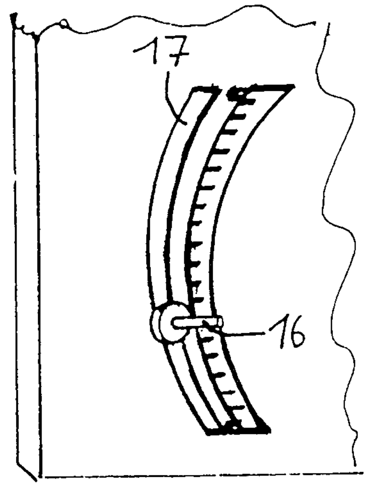


FIGUR 7

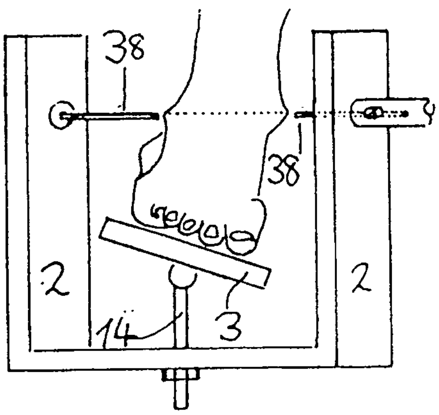


FIGUR 10

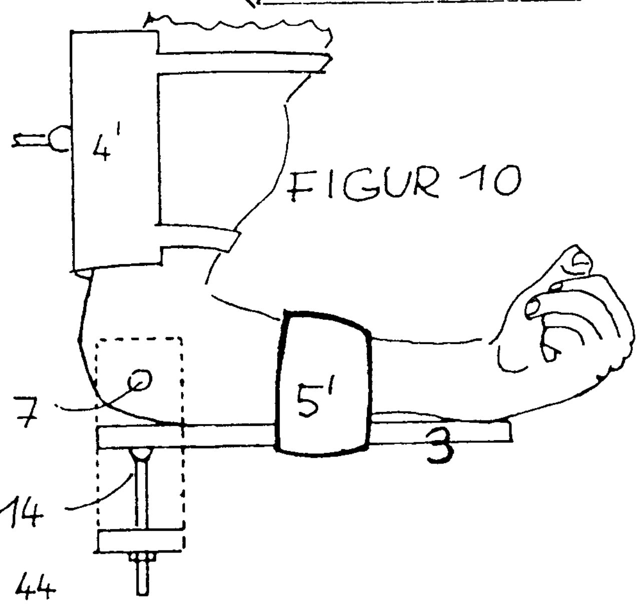


FIGUR8

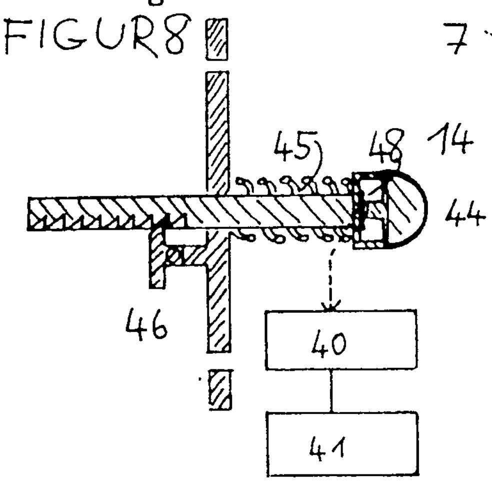

FIGUR 12

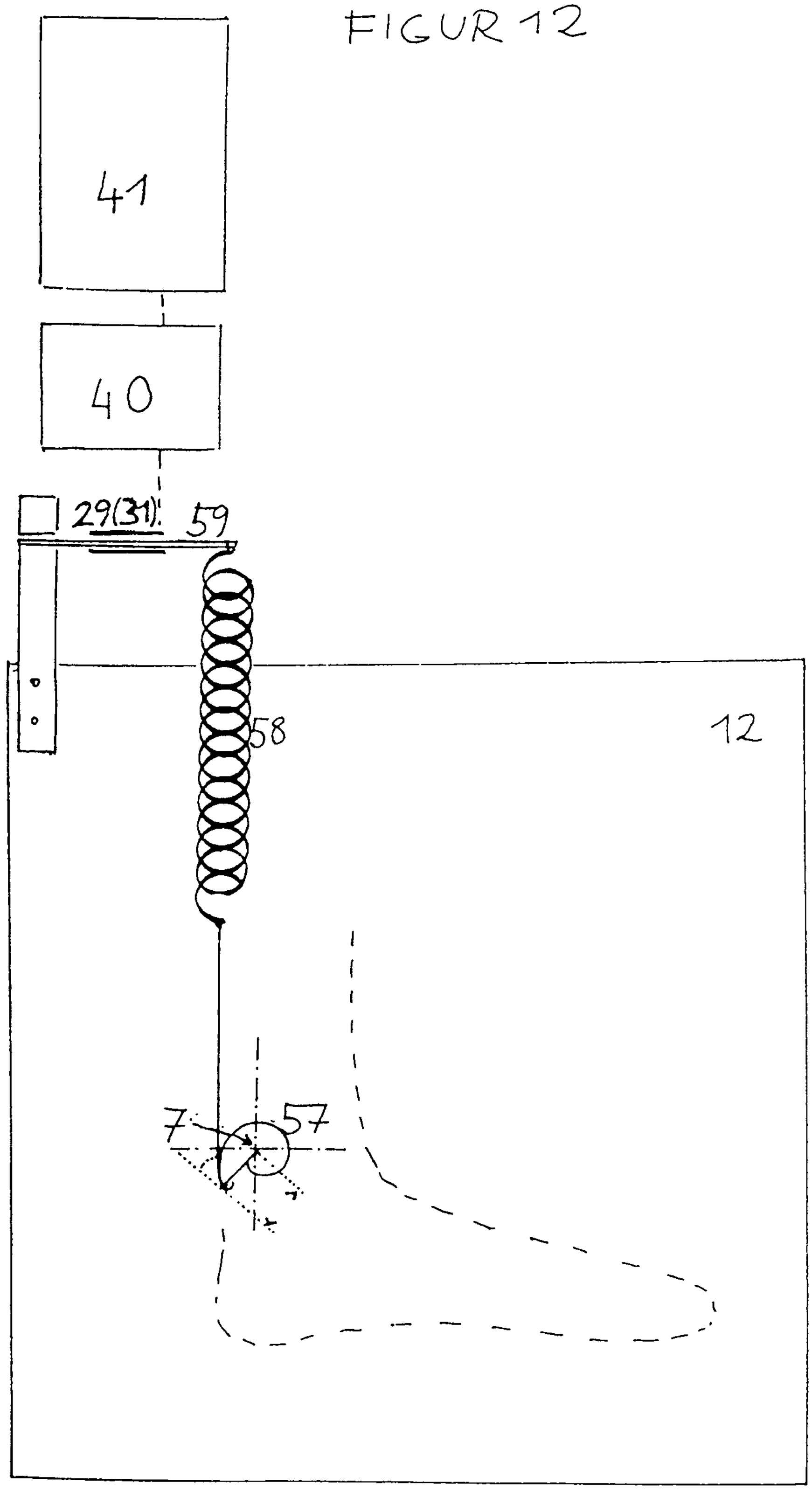

FIGUR 13

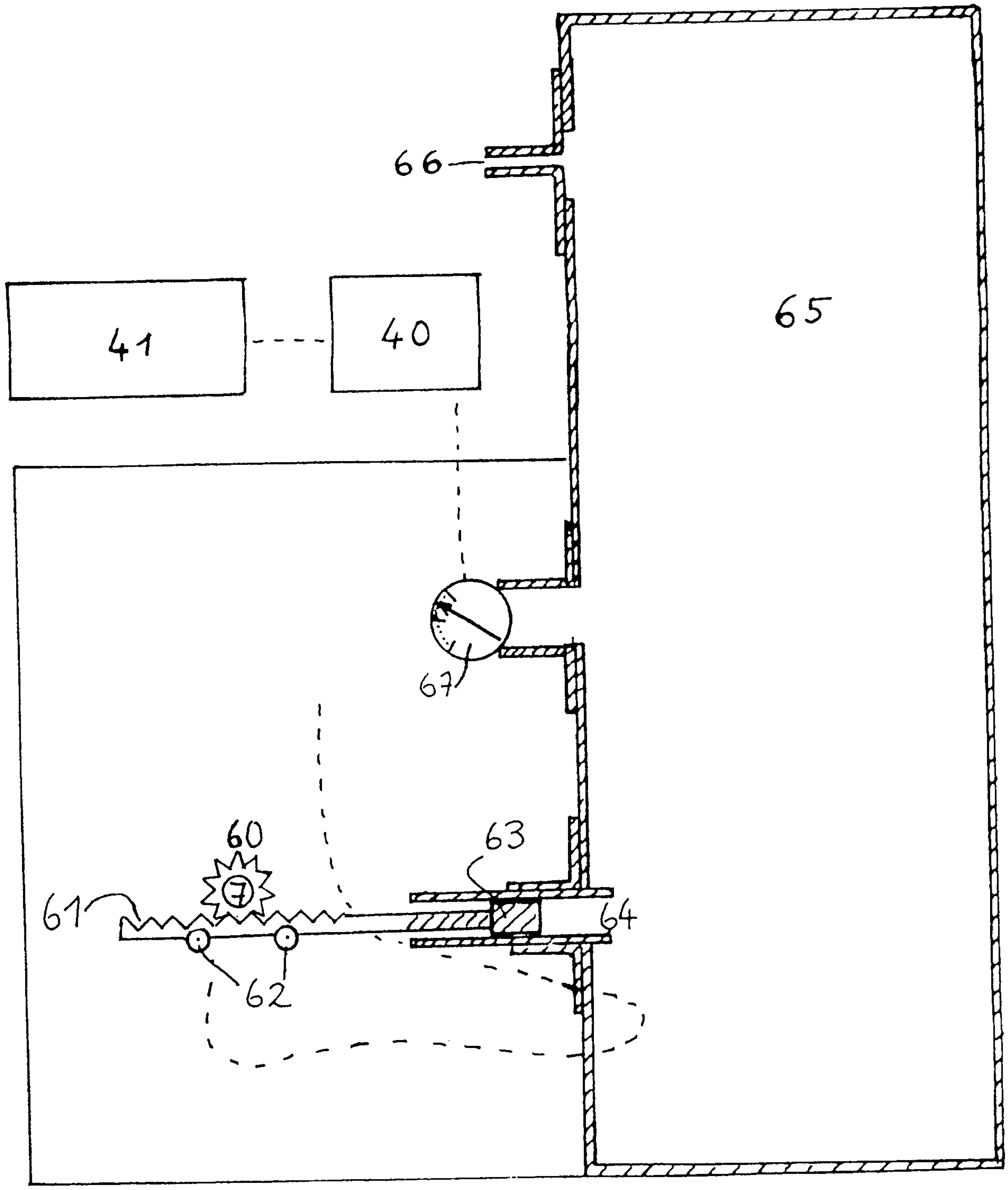

FIGUR 14

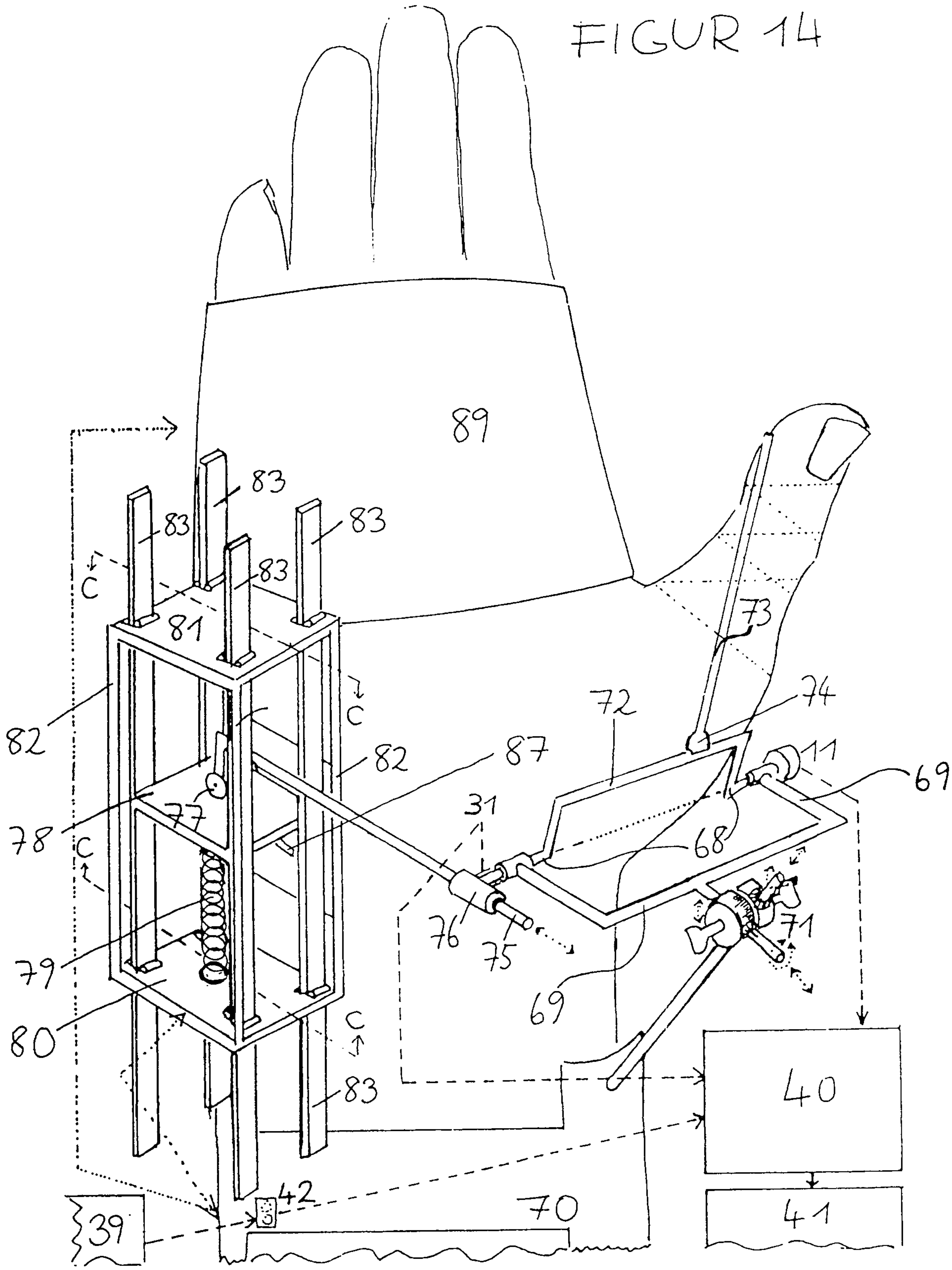
89
83
83
83
83
81
C
C
C
C
82
82
87
78
77
79
80
72
73
74
11
69
68
31
76
75
71
69
40
41
42
70
39

FIGUR 15

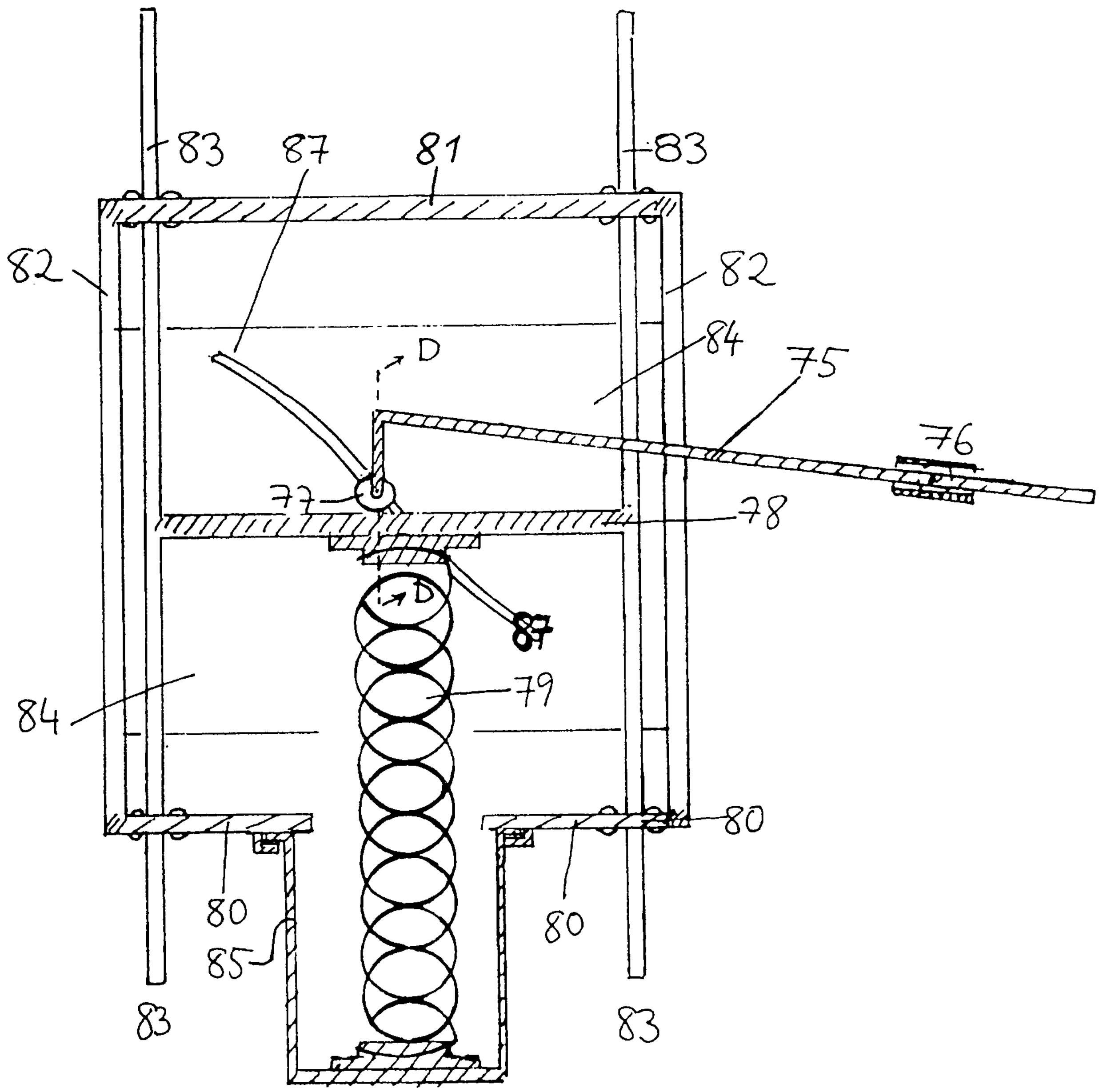

FIGUR 16

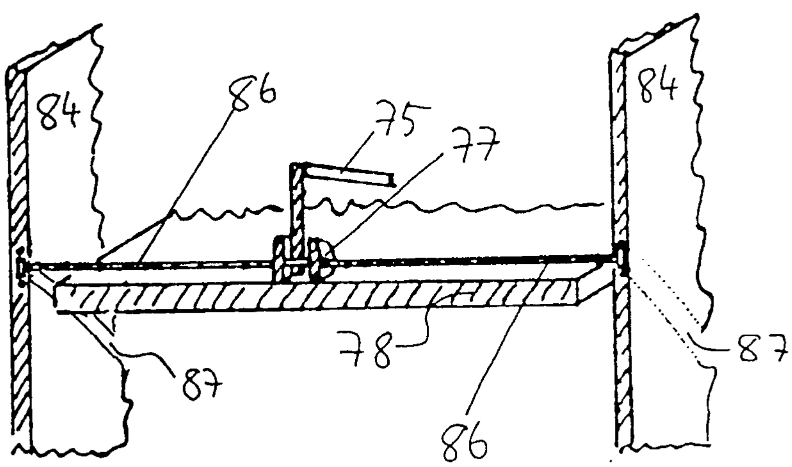

FIGUR 17

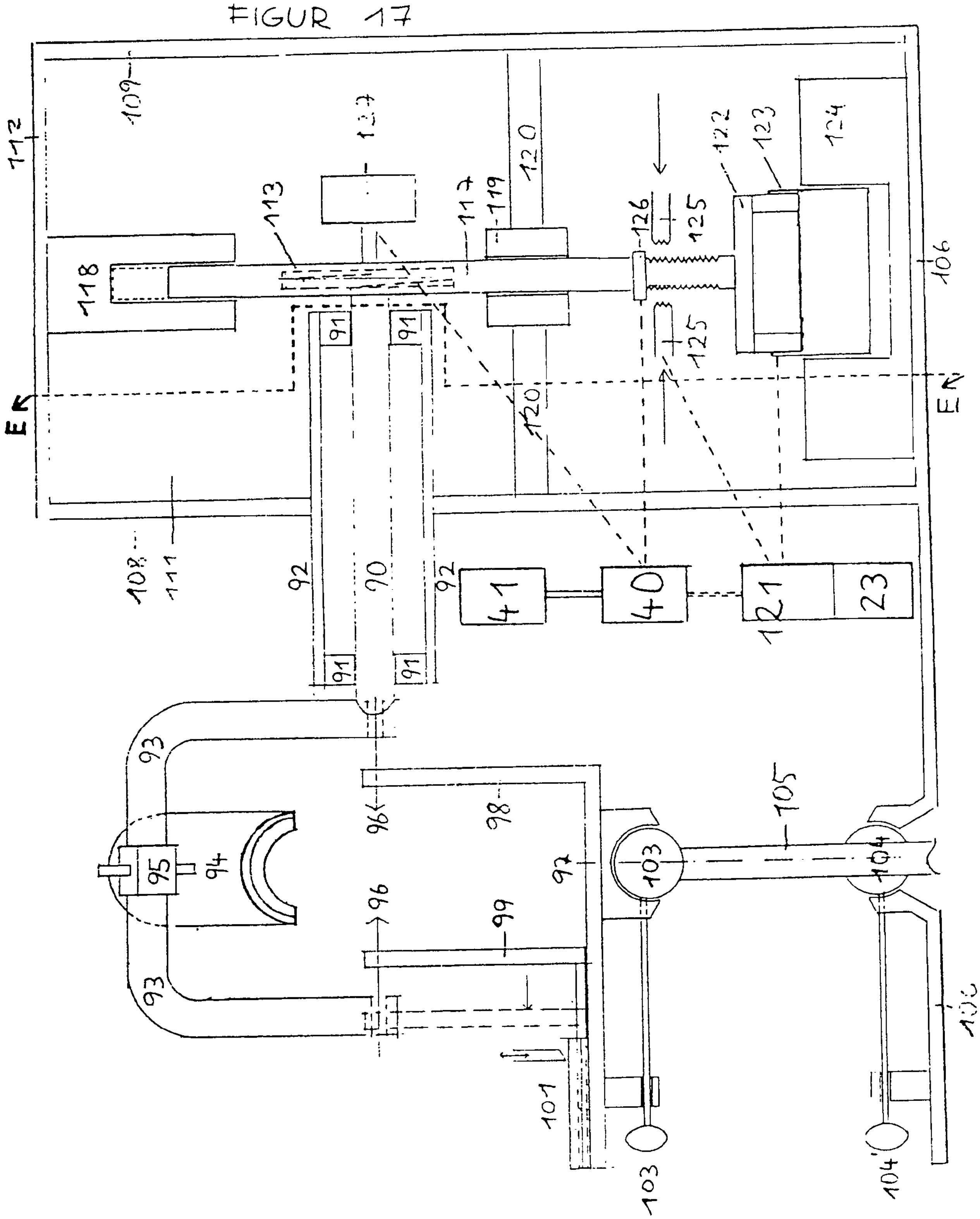

112
109
118
113
117
119
120
122
123
124
125
126
106
E
91
92
90
108
111
41
40
121
23
93
95
94
96
98
99
97
101
103
104
104'
105

FIGUR 18

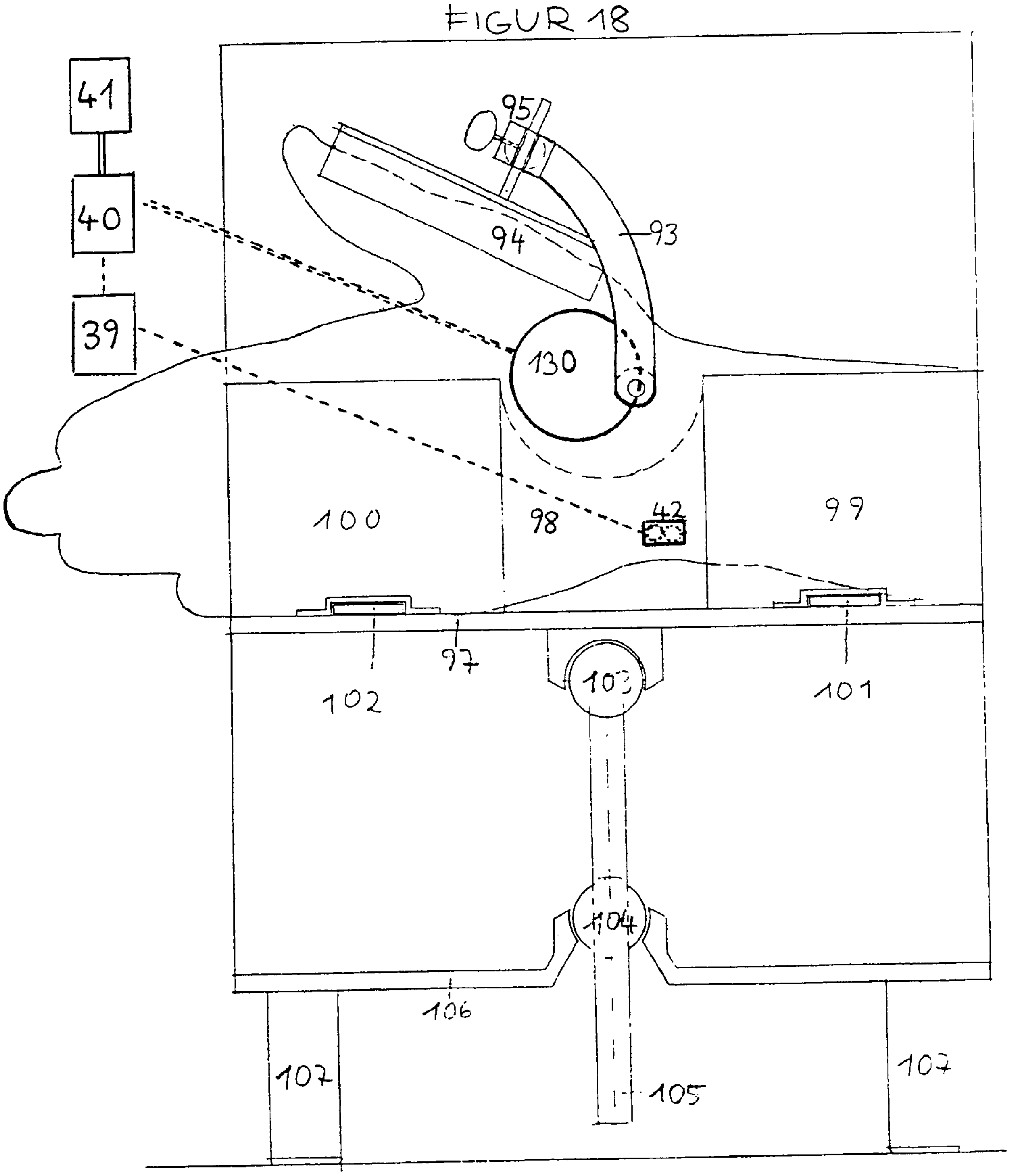
41
40
39
95
94
93
130
100
98
42
99
97
102
103
101
104
106
107
105
107

FIGUR 19

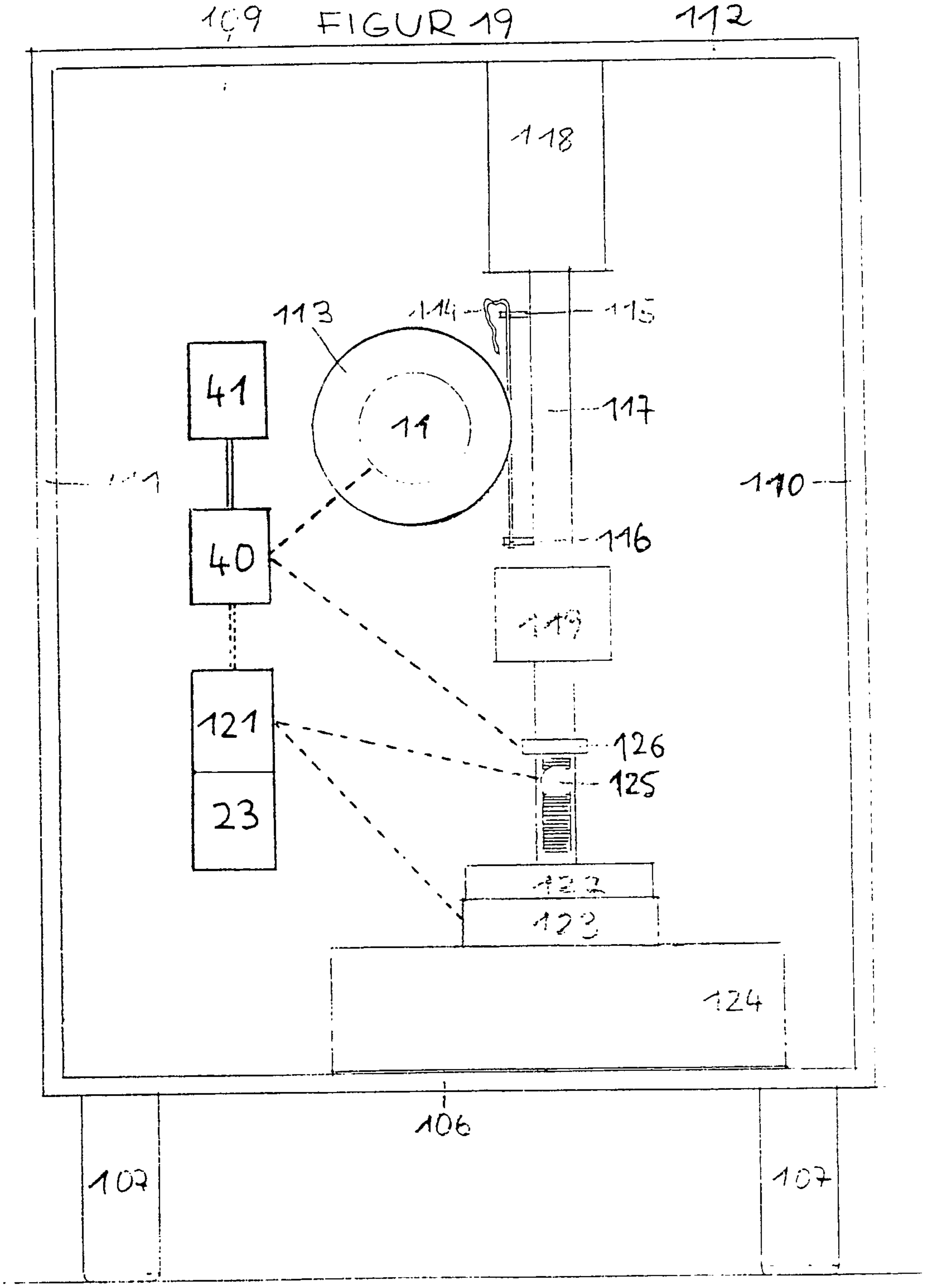
109
112
118
113
114
115
41
11
117
111
110
40
116
119
121
126
125
23
122
123
124
106
107
107

FIGUR 20

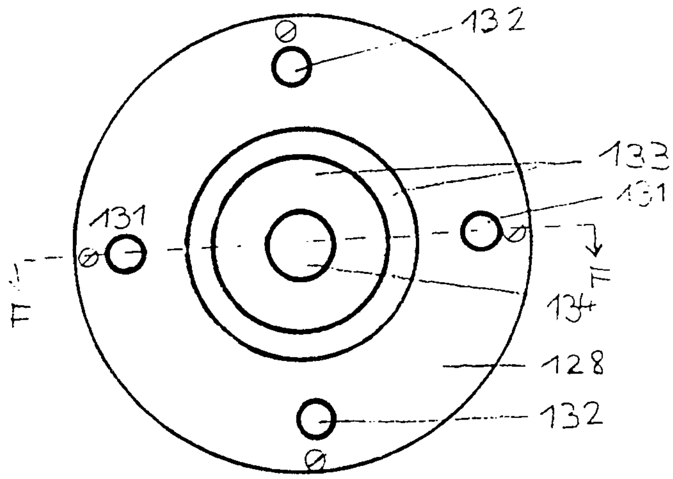


FIGUR 21